# EUROPÄISCHE PATENTSCHRIFT

(11) **EP 1 729 723 B1**
(45) Veröffentlichungstag und Bekanntmachung des Hinweises auf die Patenterteilung: **04.06.2008**
(21) Anmeldenummer: 04790861.1
(22) Anmeldetag: 26.10.2004
(51) Int. Cl.: A61K 8/40

(54) **NEUTRALE UND KATIONISCHE NAPHTHALINDERIVATIVE UND DIESE VERBINDUNGEN ENTHALTENDE FÄRBEMITTEL FÜR KERATINFASERN**
NEUTRAL AND CATIONIC NAPHTHALENE DERIVATIVES AND DYES CONTAINING SAID COMPOUNDS FOR DYEING KERATIN FIBERS
DERIVES NAPHTHALINIQUES NEUTRES ET CATIONIQUES ET MATIERES COLORANTES RENFERMANT CES COMPOSES POUR FIBRES KERATINIQUES

(30) Priorität: 07.02.2004 DE 102004006142
(43) Veröffentlichungstag der Anmeldung: 13.12.2006
(73) Patentinhaber: Wella Aktiengesellschaft, 64274 Darmstadt (DE)
(72) Erfinder: SPECKBACHER, Markus, D-63739 Aschaffenburg (DE); BRAUN, Hans-Jürgen, CH-3182 Ueberstorf (CH); CHASSOT, Jessica, CH-1693 Chavannes-sous-Orsonnens (CH)
(74) Vertreter: Bockhorst, Matthias
(86) Internationale Anmeldenummer: PCT/EP2004/012078
(87) Internationale Veröffentlichungsnummer: WO 2005/075574

(56) Entgegenhaltungen:
- EP-A- 1 464 328
- WO-A-02/066562
- DE-A1- 2 236 555
- DE-C- 887 198
- DE-C- 953 064
- FR-A- 2 853 231
- GB-A- 962 019
- GB-A- 973 260
- US-A- 2 006 017
- US-A- 5 292 881
- PLKIDIN ET AL.: "Derivatives of Naphthalic acid" J.ORG.CHEM.USSR, 1983, Seiten 2273-2281, XP009046497
- ORZESZKO ET AL.: "Investigation of the Thionation Reaction of Cyclic Imides" Z.NATURFORSCH., Bd. 56b, 2001, Seiten 1035-1040, XP009046498
- LETHAO ET AL.: "Photoinduced Electron Transfer in Covalently Linked 1,8-Naphthalimide/Viologen Systems" J.PHYS.CHEM.A, Bd. 104, 2000, Seiten 6778-6785, XP002325350
- STOLL ET AL.: "Über Derivate des Naphtostyrils" HELV.CHIM.ACTA, Bd. 41, 1951, Seiten 382-396, XP009046511
- WOJCIECHOWSKI ET AL.: "Effect of Amide groups in p-NN-Dimethylaminoazobenzene derivatives on their spectral properties wihin the UV-Vis Range" POLISH J.CHEM., Bd. 60, 1986, Seiten 797-810, XP009046479
- RICE ET AL.: "Imidothiazoles" J.MED.CHEM., Bd. 11, Nr. 1, 1968, Seiten 183-185, XP002325351
- WENDELIN ET AL.: "Fluorescence Reagents" J.HETEROCYCLIC CHEM., Bd. 24, 1987, Seiten 1381-1390, XP009046510
- GABBAY ET AL.: "Topography of Nucleic acid helices in Solution" BIOCHEM.BIOPHYS.RES.COMM., Bd. 51, Nr. 4, 1973, Seiten 1083-1089, XP009046470
- BIADASZ ET AL.: "Langmuir films of Dichroic Dyes with Fluorescent Properties" DYES AND PIGMENTS, Bd. 56, 2003, Seiten 209-217, XP002325352
- BRANA ET AL.: "Synthesis and cytostatic Activity of Benz(de)iso-quinoli- 1,3-diones" EUR.J.MED.CHEM., Bd. 16, Nr. 3, 1981, Seiten 207-212, XP002051906
- POURJAVADI ET AL.: "Selective Preparation of Fluorescent 1,8-naphthalimides Using acidic Alumina under Microwave Irradiation" J.CHEM.RES.(S), 2001, Seiten 485-487, XP009046502

## Beschreibung

Gegenstand der vorliegenden Erfindung sind neuartige ungeladene und kationische Naphthalinfarbstoffe sowie diese Verbindungen enthaltene Färbemittel für Keratinfasern, wie zum Beispiel menschliche Haare.

Für das Färben von Fasermaterialien, insbesondere von keratinhaltigen Fasern, z.B. Haaren, Wolle oder Pelzen, kommen im allgemeinen entweder Oxidationsfarbstoffe, die durch oxidative Kupplung einer oder mehrerer Entwicklerkomponenten mit einer oder mehreren Kupplerkomponenten entstehen, oder direktziehende Farbstoffe zur Anwendung. Bei Bedarf können dem oxidativen System oxidationsstabile, direktziehende Farbstoffe zugesetzt werden, um besondere Farbeffekte zu erzielen. Direktziehende Farbstoffe werden in geeignete Trägermassen eingearbeitet, um dann auf die Faser aufgebracht zu werden. Dieses Verfahren, allgemein als Tönung bekannt, ist einfach anzuwenden, ausgesprochen mild und zeichnet sich durch eine geringe Schädigung der Faser aus, da ohne den Zusatz von Ammoniak oder Peroxid gearbeitet werden kann. Die hierbei verwendeten Farbstoffe müssen allerdings einige Anforderungen erfüllen. So müssen sie in toxikologischer und dermatologischer Hinsicht unbedenklich sein und die Erzielung von Färbungen in der gewünschten Intensität und Brillianz ermöglichen. Außerdem müssen sie eine gute Waschechtheit, Lichtechtheit, Schweißechtheit, Dauerwellechtheit, Säureechtheit, Basenechtheit und Reibeechtheit aufweisen. In jedem Fall müssen solche Haarfärbungen unter den heute üblichen Alltags-bedingungen mindestens vier bis sechs. Wochen stabil bleiben.

Für ein direktziehendes, nicht-oxidatives Färbemittel für Keratinfasern wird in der Regel eine Kombination von verschiedenen nicht-oxidativen Farbstoffen benötigt, um bestimmte Nuancierungen zu erreichen. Da die Auswahl an gelben, roten und blauen Farbstoffen, die alle Anforderungen hinreichend erfüllen, beschränkt ist, besteht weiterhin ein großer Bedarf an derartigen Farbstoffen. Eine weitere, sehr interessante Anwendung für direktziehende Farbstoffe, ergibt sich bei ihrer Anwendung in Mitteln zur gleichzeitigen Aufhellung und Färbung. In diesen Färbemassen, die einen höheren Gehalt an Oxidationsmitteln besitzen können, werden noch weitergehende Anforderungen an die verwendeten Farbstoffe gestellt, insbesondere im Hinblick auf eine ausreichende Beständigkeit gegenüber den eingesetzten Oxidationsmitteln.

Bisher gibt es kaum Farbstoffe, die die vorgenannten Voraussetzungen erfüllen und gleichzeitig ein zufriedenstellendes Färbeergebnis liefern. Aufgabe der vorliegenden Erfindung ist es daher, direktziehende Farbstoffe zum Färben von Keratinfasern, insbesondere von menschlichen Haaren, bereitzustellen, die diesen Anforderungen genügen.

Überraschenderweise wurde nunmehr gefunden, dass bestimmte neuartige Naphthalinderivate der allgemeinen Formel (I) sich als direktziehende Farbstoffe sowohl in Färbemassen ohne Oxidationsmittel als auch in aufhellenden Färbemassen mit höheren Peroxid- und/oder Persulfatanteilen eingesetzt werden können. Zudem sind die erfindungsgemäßen Farbstoffe enthaltende Färbemittel in ihren färberischen Eigenschaften üblichen Färbemitteln überlegen.

Gegenstand der vorliegenden Erfindung sind daher Naphthalinderivate der allgemeinen Formel (1) enthaltende Mittel zum Färben von Keratinfasern, 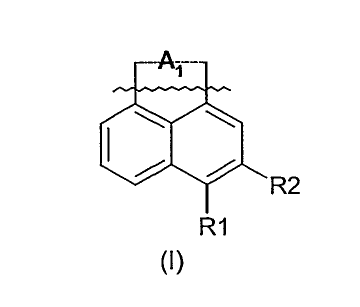 worin
**A₁** eine Teilstruktur der Formeln (II), (III), (IV), (V) und (VI) darstellt, 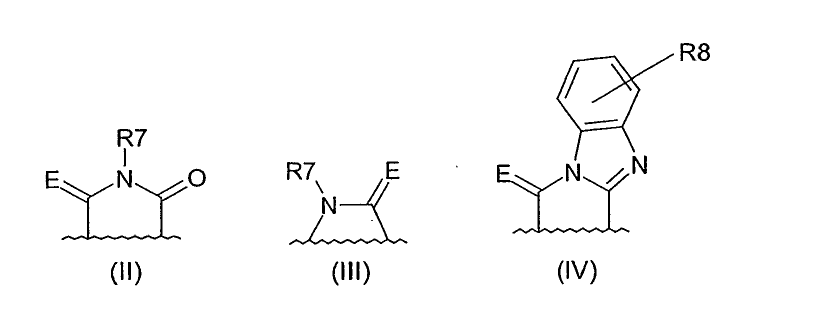 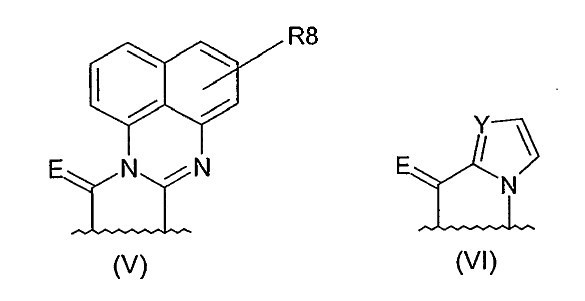 darstellt;
**E** ein Sauerstoff oder ein Schwefelatom darstellt;
**Y** für ein Stickstoffatom oder (vorzugsweise) ein quaternäres Stickstoffatom steht, welches mit verzweigten oder linearen C₁-C₆-Alkylgruppen, verzweigten oder linearen C₂-C₄-Hydroxyalkylgruppen oder verzweigten oder linearen C₄-C₆-Polyhydroxyalkylgruppen substituiert ist;
**R₁** und **R₂** unabhängig voneinander für Wasserstoff, eine -N-R₃R₄-Gruppe, eine Nitrogruppe, eine -N=N-R₅-Gruppe oder eine -N=C-R₃R₆-Gruppe stehen;
**R₃** und **R₄** gleich oder verschieden sein können und Wasserstoff, eine C₁-C₆-Alkylaminogruppe, eine C₁-C₆-N,N-Dialkylaminogruppe, eine C₁-C₆-Alkylcyanogruppe, eine Methoxymethylgruppe, eine tert-Butylgruppe, eine Isopropylgruppe, eine C₁-C₆-Alkylgruppe, eine C₁-C₆-Alkyloxygruppe, eine C₁-C₆-Hydroxyalkylgruppe, eine C₁-C₆-Alkylcarbonsäuregruppe, eine C₁-C₆-Alkylcarbonsäureestergruppe, eine C₁-C₆-Alkylcarbonsäureamidgruppe, eine C₁-C₆-Alkylsulfonsäuregruppe, eine C₁-C₆-Alkylsulfonsäureestergruppe, eine C₁-C₆-Alkylsulfonsäureamidgruppe, eine Phenylgruppe oder eine -(L)-B⁺-Gruppe darstellen;
**R₅** eine Gruppe der Formel (VII) oder (VIII) darstellt, wobei ein Rest der Formel (VIII) besonders bevorzugt ist; 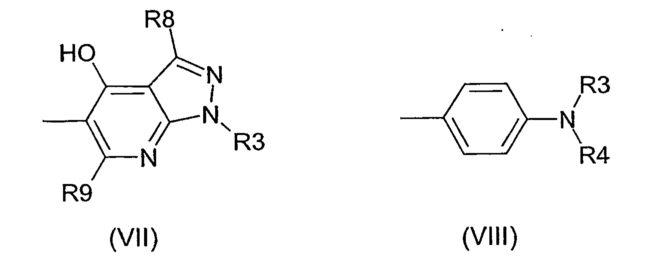
**R₆** eine Verbindung der Formel (IX), (X) oder (XI) darstellt; 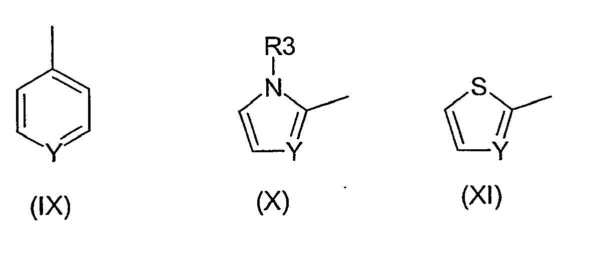
**R₇** ein Wasserstoffatom, einen aliphatischen C₁-C₆-Alkylrest, der linear oder verzweigt, unsubstituiert oder mit einer oder mehreren Hydroxylgruppen oder kationischen Resten des Typs B⁺ substituiert ist; einen aromatischen Rest der allgemeinen Formel (XII) oder (XIII); 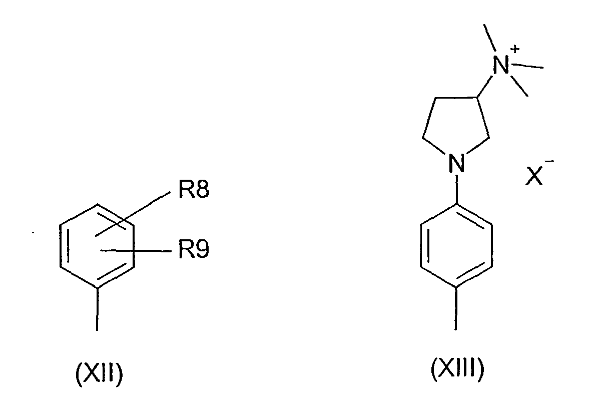 oder einen fünfgliedrigen oder sechsgliedrigen Heterozyklus oder (vorzugsweise) heterozyklische Bizyklus der allgemeinen Formeln (XIV), (XV), (XVI) oder (XVII) darstellt; 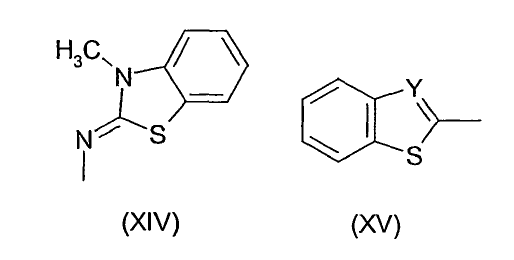 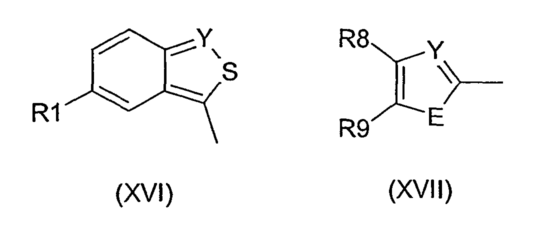
**R₈** und **R₉** gleich oder verschieden sein können und Wasserstoff, eine Aminogruppe, eine C₁-C₆-Alkylaminogruppe, eine C₁-C₆-N,N-Dialkylaminogruppe, eine C₁-C₆-N,N-(Dihydroxyalkyl)aminogruppe, Fluor, Chlor, Brom, Jod, eine Cyanogruppe, eine C₁-C₆-Alkylcyanogruppe, eine Methoxymethylgruppe, eine tert-Butylgruppe, eine Isopropylgruppe, eine C₁-C₆-Alkylgruppe, eine C₁-C₆-Alkyloxygruppe, eine C₁-C₆-Hydroxyalkylgruppe, eine C₁-C₆-Hydroxyalkyloxygruppe, eine C₁-C₆-Alkylcarbonsäuregruppe, eine C₁-C₆-Alkylcarbonsäureestergruppe, eine C₁-C₆-Alkylcarbonsäureamidgruppe, eine C₁-C₆-Alkylsulfonsäuregruppe, eine C₁-C₆-Alkylsulfonsäureestergruppe, eine C₁-C₆-Alkylsulfonsäureamidgruppe, eine Phenylgruppe, eine Sulfonsäuregruppe oder eine -(L)-B⁺-Gruppe darstellen;
**L** für eine C₁-C₆-Alkylengruppe steht;
**B⁺** für eine aromatische heterozyklische quaternäre Ammoniumverbindung -vorzugsweise eine quaternäre Verbindung des N-Methylimidazols, N-Allylimidazols, 2-Ethylimidazols, 1,2-Dimethylimidazols, Pyridins, 4-Dimethylaminopyridins, Pyrimidins, Pyrazols, N-Methylpyrazols oder Chinolins-; eine nicht-aromatische heterozyklische quaternäre Ammoniumverbindung -insbesondere eine quaternäre Verbindung des N-Methyl-morpholins, N-Ethylmorpholins oder 1-Methylpiperidins-; eine quaternäre Alkylammoniumverbindung oder Arylammoniumverbindung der Formel NRₐR_{b}R_{c}, wobei **Rₐ, R_{b}**, und **R_{c}** unabhängig voneinander einen Benzylrest, einen Phenylrest oder einen C₁-bis C₆-Alkylrest -insbesondere einen Methylrest, einen Ethylrest, einen Propylrest, einen Isopropylrest oder einen Butylrest- bedeuten, wobei die vorgenannten Alkylreste unsubstituiert oder mit einer oder mehreren Hydroxylgruppen oder Aminogruppen substituiert sein können; oder eine quaternäre Phosphoniumgruppe, beispielsweise eine Tributylphosphoniumgruppe, insbesondere aber eine Trimethylammoniumgruppe oder eine Triethylammoniumgruppe, steht; und
**X⁻** ein Anion, vorzugsweise ein Sulfatanion, ein Phosphatanion, ein Hydrogenphosphatanion, ein Oxalatanion, ein Formiatanion, ein Acetatanion, ein Zitratanion, ein Tartratanion, ein Malonatanion, ein Pyruvatanion, ein Chloridanion, ein Bromidanion, ein Iodidanion oder ein Methylsulfatanion darstellt, wobei das Chloridanion, das Bromidanion und das Methylsulfatanion besonders bevorzugt sind.

Als geeignete neutrale oder kationische Direktzieher der allgemeinen Formel (I) können beispielsweise genannt werden: 3-((E)-{4-[Ethyl(2-hydroxyethyl)amino]phenyl}diazenyl)-8-methyl-7-oxo-7H-benzo[de]-imidazo-[1,2-a]chinolin-8-ium-methylsulfat, 3-{(E)-[4-Hydroxy-1-(2-hydroxyethyl)-3,6-dimethyl-1 H-pyrazolo[3,4-b]pyridin-5-yl]diazenyl}-8-methyl-7-oxo-7H-benzo[de]imidazo[1,2-a]chinolin-8-ium-methylsulfat, 3-Methyl-2-(5-nitro-2-oxobenzo[cd]indol-1 (2H)-yl)-1,3-benzothiazol-3-ium-methylsulfat, 3-Nitro-7H-benzo[de]imidazo[1,2-a]chinolin-7-on, 2-(5-(Dimethylamino)-2-oxobenzo[cd]indol-1 (2H)-yl)-3-methyl-1,3-benzothiazol-3-ium-methylsulfat, 3-{2-[4-(5-(Dimethylamino)-1,3-dioxo-1H-benzo[de]isochinolin-2(3H)-yl)(ethyl)anilino]ethyl}-1-methyl-1H-imidazol-3-ium-bromid, 5-((E)-{1-[4-(Dimethylamino)phenyl]-2-oxo-1,2-dihydrobenzo-[cd]indol-6-yl}diazenyl)-4-hydroxy-1-(2-hydroxyethyl)-3,6,7-trimethyl-1H-pyrazolo[3,4-b]pyridin-7-ium-methylsulfat, 3-(Dimethylamino)-8-methyl-7-oxo-7H-benzo[de]imidazo[1,2-a]chinolin-8-ium-methylsulfat, 1-{4-[bis(Hydroxyethyl)amino]phenyl}-6-nitrobenzo[cd]indol-2(1H)-on, 3-{2-[Ethyl-4-(6-nitro-2-oxobenzo[cd]indol-1 (2H)-yl)anilino]ethyl}-1-methyl-1 H-imidazol-3-ium-bromid, 4-((E)-{[2-(3,4-Dimethoxyphenyl)-1,3-dioxo-2,3-dihydro-1 H-benzo[de]isoquinolin-6-yl]imino}methyl)-1-(2-hydroxyethyl)-pyridinium-bromid, 2-(3,4-Dimethoxyphenyl)-6-nitro-1 H-benzo[de]-isochinolin-1,3(2H)-dion, 1-(3,4-Dimethoxyphenyl)-6-nitrobenzo-[cd]indol-2(1H)-on, 1-Methyl-3-[2-({2-[((2Z)-3-methyl-1,3-benzthiazol-2(3H)-ylidene)amino]-1,3-dioxo-2,3-dihydro-1 H-benzo[de]isochinolin-6-yl}amino)ethyl]-1 H-imidazol-3-ium-bromid, 1-(2-Hydroxyethyl)-4-{(E)-[(7-oxo-7H-benzimidazo[2,1-a]benzo[de]isochuinolin-2-yl)imino]methyl}-pyridinium-bromid, 1-(2-Hydroxyethyl-4-{(E)-[7-thioxo-7H-benzimidazo[2,1-a]benzo[de]isochinolin-2-yl)imino]methyl}-pyridinium-bromid, 2-{Ethyl-4-[(E)-(14-oxo-14H-benzo[4,5]isochinolino[2,1-a]perimidin-9-yl)diazenyl]-anilino}-N,N,N-trimethylethylethanaminium-bromid, 2-{Ethyl-4-[(E)-(7-oxo-7H-benzimidazo[2,1-a]benzo[de]isochinolin-2-yl)diazenyl]anilino}-N,N,N-trimethylethanaminium-bromid, 2-{Ethyl-4-[(E)-(7-thioxo-7H-benzimidazo-[2,1-a]benzo[de]isochinolin-2-yl)diazenyl]anilino}-N,N,N-trimethylethanaminium-bromid, 2-[4-[(E)-(1,3-dioxo-2,3-dihydro-1 H-benzo[de]isochinolin-6-yl)diazenyl](ethyl)anilino]-N,N,N-trimethylethanaminium-bromid, 1-(2-Hydroxyethyl)-4-[(E)-({2-[((2Z)-3-methyl-1,3-benzothiazol-2(3H)-ylidene)amino]-1,3-dioxo-2,3-dihydro-1 H-benzo[de]isochinolin-5-yl}imino)-methyl]pyridinium-bromid, 4-{(E)-[(1,3-Dioxo-2,3-dihydro-1H-benzo[de]-isochinolin-6-yl)imino]methyl}-1-(2-hydroxyethyl)pyridinium-bromid, 2-[4-((E)-{1-[4-(Dimethylamino)phenyl]-2-oxo-1,2-dihydrobenzo[cd]indol-5-yl}diazenyl)(ethyl)anilino]-N,N,N-trimethylethanaminium-bromid, 3-{2-[Ethyl-4-(2-oxo-5-((E)-{3,4,6-trimethyl-1-[2-(1-methyl-1H-imidazol-3-ium-3-yl)ethyl-1H-pyrazolo[3,4-b]pyridin-5-yl}diazenyl)benzo[cd]indol-1 (2H)-yl)anilino]ethyl}-1-methyl-1H-imidazol-3-ium-dibromid, 1-(2-Hydroxyethyl)-4-[(E)-({2-[((2Z)-3-methyl-1,3-benzothiazol-2(3H)-ylidene)amino]-1,3-dioxo-2,3-dihydro-1H-benzo[de]isochinolin-6-yl}imino)methyl]pyridinium-bromid, 2-(6-(Dimethylamino)-1,3-dioxo-1H-benzo[de]isoquinolin-2(3H)-yl)-3,4,5-trimethyl-1,3-thiazol-3-ium-methylsulfat, 2-(5-(Dimethylamino)-2-oxobenzo[cd]indol-1(2H)-yl)-3,4,5-trimethyl-1,3-thiazol-3-ium-methylsulfat, 3-{2-[Ethyl-4-(6-nitro-1,3-dioxo-1H-benzo[de]isochinolin-2(3H)-yl)anilino]ethyl}-1-methyl-1H-imidazol-3-ium-bromid, 3-{2-[5-Methoxy-2-(6-nitro-1,3-dioxo-1H-benzo[de]isochinolin-2(3H)-yl)anilino]ethyl}-1-methyl-1 H-imidazol-3-ium-bromid, 2-[(3-methyl-1,3-benzothiazol-2(3H)-ylidene)amino]-6-nitro-1 H-benzo[de]isochinolin-1,3(2H)-dion, 2-(1,3-Benzothiazol-2-yl)-6-nitro-1H-benzo[de]isochinolin-1,3(2H)-dion, 1-(2-Hydroxyethyl)-4-{(E)-[(14-oxo-14H-benzo[4,5]isochino[2,1-a]perimidin-9-yl)imino]methyl}pyridinium-bromid und 2-{2-[(2-Hydroxyethyl)amino]-4-methoxypheny)}-5-nitro-1 H-benzo[de]isochinolin-1,3(2H)-dion.

Bevorzugte Verbindungen der allgemeinen Formel (1) sind 3-((E)-{4-[Ethyl(2-hydroxyethyl)amino]phenyl}diazenyl)-8-methyl-7-oxo-7H-benzo[de]imidazo-[1,2-a]chinolin-8-ium-methylsulfat, 3-{(E)-[4-Hydroxy-1-(2-hydroxyethyl)-3,6-dimethyl-1 H-pyrazolo[3,4-b]pyridin-5-yl]diazenyl}-8-methyl-7-oxo-7H-benzo[de]imidazo[1,2-a]quinolin-8-ium-methylsulfat, 3-Methyl-2-(5-nitro-2-oxobenzo[cd]indol-1(2H)-yl)-1,3-benzothiazol-3-ium-methylsulfat, 2-(5-(Dimethylamino)-2-oxobenzo[cd]indol-1 (2H)-yl)-3-methyl-1,3-benzothiazol-3-ium-methylsulfat, 5-((E)-{1-[4-(Dimethylamino)-phenyl]-2-oxo-1,2-dihydrobenzo[cd]indol-6-yl}diazenyl)-4-hydroxy-1-(2-hydroxyethyl)-3,6,7-trimethyl-1 H-pyrazolo[3,4-b]pyridin-7-ium-methylsulfat, 1-{4-[bis(Hydroxyethyl)amino]phenyl}-6-nitrobenzo[cd]indol-2(1H)-on, 3-{2-[Ethyl-4-(6-nitro-2-oxobenzo[cd]indol-1 (2H)-yl)anilino]ethyl}-1-methyl-1 H-imidazol-3-ium-bromid, 1-(2-Hydroxyethyl)-4-{(E)-[(7-oxo-7H-benzimidazo[2,1-a]benzo[de]isochinolin-2-yl)imino]methyl}pyridinium-bromid, 4-{(E)-[(1,3-Dioxo-2,3-dihydro-1H-benzo[de]isochinolin-6-yl)imino]methyl}-1-(2-hydroxyethyl)pyridinium-bromid, 2-[4-((E)-{1-[4-(Dimethylamino)phenyl]-2-oxo-1,2-dihydrobenzo[cd]indol-5-yl}diazenyl)-(ethyl)anilino]-N,N,N-trimethylethanaminium-bromid, 2-[(3-Methyl-1,3-benzothiazol-2(3H)-ylidene)amino]-6-nitro-1 H-benzo[de]isochinolin-1,3(2H)-dion, 2-(1,3-Benzothiazol-2-yl)-6-nitro-1H-benzo[de]isochinolin-1,3(2H)-dion, 1-(2-Hydroxyethyl)-4-{(E)-[(14-oxo-14H-benzo[4,5]isoquino-[2,1-a]perimidin-9-yl)imino]methyl}pyridinium-bromid und 2-{2-[(2-Hydroxyethyl)amino]-4-methoxyphenyl}-5-nitro-1H-benzo[de]isochinolin-1,3(2H)-dion.

Die erfindungsgemässen Naphthalinderivate der allgemeinen Formel (I) sind durch bekannte Syntheseverfahren aus kommerziell erhältlichen oder leicht herstellbaren Komponenten zugänglich.

Als Naphthalinvorstufen der allgemeinen Formel (XVIII) können beispielsweise die folgenden Anhydride eingesetzt werden: 4-Nitro-naphthalin-1,8-dicarbonsäureanhydrid und 3-Nitro-naphthalin-1,8-dicarbonsäureanhydrid.

Durch Kondensationsreaktionen bei erhöhter Temperatur in geeigneten Lösungsmitteln, wie zum Beispiel Eisessig, DMF oder geschmolzenem Imidazol, lassen sich gemäß Schema 1 aus Naphthalin-1,8-dicarbonsäureanhydrid der Formel (XVIII) mit primären aliphatischen, aromatischen oder heterozyklischen Aminen sowie Hydrazonen (A1) die entsprechenden unsymmetrisch substituierten Imide, Amidine oder Isoamidine herstellen.

Lactame (XX) lassen sich nach H. Langhals et. al. (Angew. Chem. 1995, 107, 2436-2439; Angew. Chem., Int. Ed. Engl. 1995, 34, 2234-2236) und EP 0 769 532 A1 durch eine Ringverengungsreaktion in einer DMSO/Methanol-Mischung unter stark alkalischen Bedingungen aus beliebigen Bisimid-Derivaten (XIX) darstellen (Schema 2).

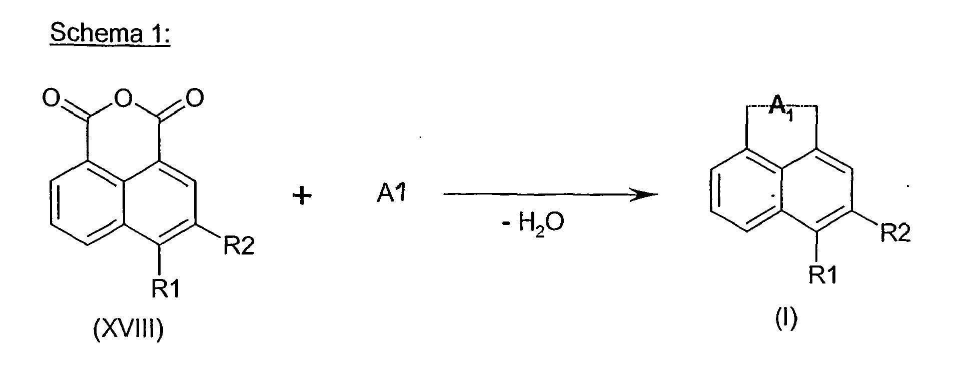

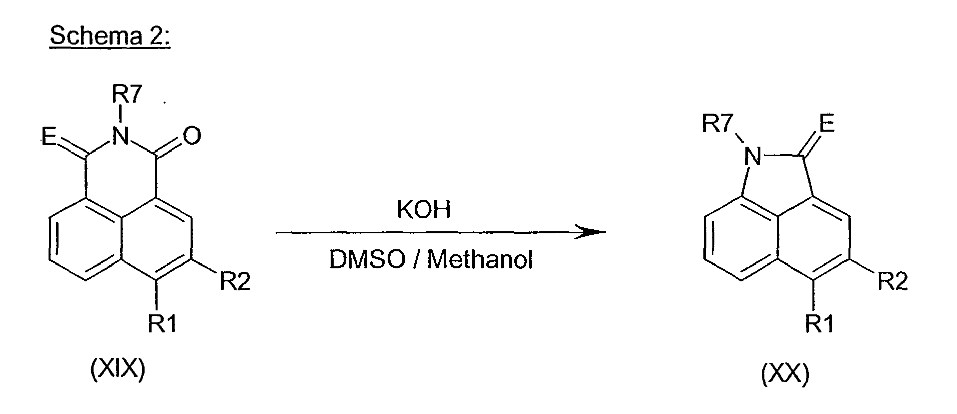

Die kationischen Vertreter sind auf einfache Weise entweder durch Einführung einer kationischen Gruppe (Schema 3) oder durch Quaternisierung von heterozyklischen Stickstoffatomen (Schema 45) darstellbar.

Q-L-Hal + B → Q-L-B⁺ + Hal⁻ Schema 3:

Gemäß Schema 3 werden Verbindungen der allgemeinen Formel **Q-L-Hal,** worin die Restgruppe **Q** für ein neutrales Naphthalinderivat der Formel (I) und **L** für C₁-C₆-Alkyl steht, (**Hal** bedeutet Chlor, Brom oder Jod) durch nucleophile Substitution in dipolar aprotischen Lösungsmitteln mit Verbindungen des Typs **B** umgesetzt, wobei **B** für eine aromatische, heterozyklische Verbindung -vorzugsweise ein N-Methylimidazol, N-Allylimidazol, 2-Ethylimidazol oder 1,2-Dimethyl-imidazol, Pyridin, 4-Dimethylaminopyridin, Pyrimidin, Pyrazol, N-Methyl-pyrazol oder Chinolin; oder eine nicht-aromatische heterozyklische Verbindung -insbesondere ein N-Methyl-morpholin, N-Ethylmorpholin oderl-Methylpiperidin; oder eine Alkyl- oder Arylverbindung der Formel NRₐR_{b}R_{c}, wobei **Rₐ, R_{b},** und **R_{c}** die vorstehend genannte Bedeutung haben, zum Beispiel eine Trimethylaminogruppe, eine Triethylaminogruppe oder eine Tributylaminogruppe; oder eine tertiäre phosphororganische Gruppe ("tertiäres Phosphin") steht.

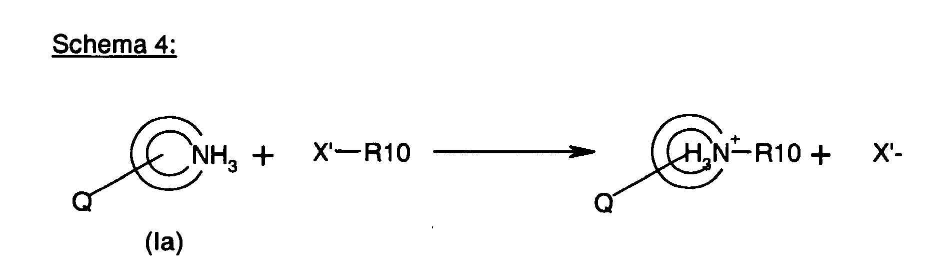

Gemäß Schema 4 werden heterozyklische Stickstoffatome in den entsprechenden neutralen Naphthalinderivaten der Formel (Ia) mit Alkylierungsmitteln der allgemeinen Formel **X'-R₁₀**, worin **X'** für Chlor, Brom, Jod oder Methylsulfat und **R₁₀** für C₁-C₆-Alkylgruppen, C₁-C₃-Hydroxyalkylgruppen oder C₄-C₆-Polyhydroxyalkylgruppen steht, quaternisiert.

Die Naphthalinderivate der allgemeinen Formel (I) sind in dem erfindungsgemäßen Färbemitteln vorzugsweise in einer Menge von 0,01 bis 10 Gewichtsprozent, insbesondere 0,1 bis 8 Gewichtsprozent, enthalten.

Das erfindungsgemäße Färbemittel (a) kann neben den Farbstoffen der allgemeinen Formel (I) zusätzlich noch weitere bekannte direktfärbende Farbstoffe aus der Gruppe bestehend aus Nitrofarbstoffen, Azofarbstoffen, Athrachinonfarbstoffen und Triphenylmethanfarbstoffen, alleine oder im Gemisch miteinander, enthalten, wie zum Beispiel 1,4-Bis[(2-hydroxyethyl)amino]-2-nitrobenzol, 1-(2-Hydroxyethyl)amino-2-nitro-4-[di(2-hydroxyethyl)amino]-benzol, (HC Blue No. 2), 1-Amino-3-methyl-4-[(2-hydroxyethyl)amino]-6-nitrobenzol, (HC Violet No. 1), 4-[Ethyl-(2-hyd roxyethyl)amino]-1-[(2-hydroxyethyl)amino]-2-nitrobenzolhydrochlorid (HC Blue No. 12), 1-[(2,3-Dihydroxypropyl)amino]-4-[methyl-(2-hydroxyethyl)amino]-2-nitrobenzol (HC Blue No. 10), 1-[(2,3-Dihydroxypropyl)amino]-4-[ethyl-(2-hydroxyethyl)amino]-2-nitrobenzolhydrochlorid (HC Blue No. 9), 1-(3-Hydroxypropylamino)-4-[di(2-hydroxyethyl)amino]-2-nitrobenzol, (HC Violet No. 2); 1-Amino-4-[(2-hydroxyethyl)amino]-2-nitrobenzol (HC Red No. 7), 2-Amino-4,6-dinitrophenol, 1,4-Diamino-2-nitrobenzol (CI76070), 4-Amino-2-nitrodiphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-2-nitro-1-((prop-2-en-1-yl)amino)-benzol, 4-Amino-3-nitrophenol, 4-[(2-Hydroxyethyl)amino]-3-nitrophenol, 4-,[(2-Nitrophenyl)amino]phenol (HC Orange No. 1), 1-[(2-Aminoethyl)-amino]-4-(2-hydroxyethoxy)-2-nitrobenzol (HC Orange No. 2), 4-(2,3-Dihydroxypropoxy)-1-[(2-hydroxyethyl)amino]-2-nitrobenzol, (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] - 2-nitrobenzol (HC Red No. 11), 2-[(2-Hydroxyethyl)amino]-4,6-dinitrophenol, 4-Ethylamino-3-nitrobenzoesäure, 2-[(4-Amino-2-nitrophenyl)-amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 3-Amino-6-(methylamino)-2-nitropyridin, 1,2,3,4-Tetrahydro-6-nitrochinoxalin, 7-Amino-3,4-dihydro-6-nitro-2H-1,4-benzoxazin (HC Red No. 14), 1,2-Diamino-4-nitrobenzol (C176020), 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol, (HC Yellow No. 4), 1-[(2-Hydroxyethyl)amino]-2-nitrobenzol (HC Yellow No. 2), 2-[(2-Hydroxyethyl)amino]-1-methoxy-5-nitrobenzol, 2-Amino-3-nitrophenol, 1-Amino-2-methyl-6-nitrobenzol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid, (HC Yellow No.9), 1-[(2-Ureidoethyl)amino]-4-nitrobenzol, 4-[(2,3-Dihydroxypropyl)amino]-3-nitro-1-trifluormethyl-benzol, (HC Yellow No. 6), 1-Chlor-2,4-bis[(2-hydroxyethyl)amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol, (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), 2,4-Dinitro-1-hydroxy-naphthalin; 1,4-Di[(2,3-dihydroxypropyl)amino]-9,10-anthrachinon, 1,4-Di[(2-hydroxyethyl)amino]-9,10-anthrachinon (CI61545, Disperse Blue 23), 1-Amino-4-hydroxy-9,10-anthrachinon (CI60710, Disperse Red 15), 1-Hydroxy-4-[(4-methyl-2-sulfophenyl)amino]-9,10-anthrachinon, 7-Beta-D-glucopyranosyl-9,10-dihydro-1-methyl-9,10-dioxo-3,5,6,8-tetrahydroxy-2-anthracencarbonsäure (CI75470, Natural Red 4), 1-[(3-Aminopropyl)amino]-9,10-anthrachinon (HC Red No. 8), 1,4-Diamino-9,10-anthrachinon (CI61100, Disperse Violet No. 1), 1-Amino-4-(methylamino)-9,10-anthrachinon (CI61105, Disperse Violet No. 4, Solvent Violet No. 12), N-(6-((3-Chlor-4-(methylamino)phenyl)-imino)-4-methyl-3-oxo-1,4-cyclohexadien-1-yl)harnstoff (HC Red No. 9), 2-((4-(Di(2-hydroxyethyl)amino)phenyl)amino)-5-((2-hydroxyethyl)amino)-2,5-cyclohexadien-1,4-dion (HC Green No. 1), 2-Hydroxy-1,4-naphthochinon (C175480, Natural Orange No. 6), 1,2-Dihydro-2-(1,3-dihydro-3-oxo-2H-indol-2-yliden)-3H-indol-3-on (C173000), 1,3-Bis-(dicyanomethylen)indan; Di[4-(diethylamino)phenyl][4-(ethylamino)-naphthyl]carbenium-chlorid (C142595; Basic Blue No. 7), Di[4-(dimethylamino)phenyl][4-(phenylamino)naphthyl]carbenium-chlorid (Cl44045; Basic Blue No. 26), Basic Blue No. 77, 8-Amino-2-brom-5-hydroxy-4-imino-6-[(3-(trimethylammonio)phenyl)amino]-1 (4H)-naphthalinon-chlorid (CI56059; Basic Blue No. 99), Tri(4-amino-3-methylphenyl)carbenium-chlorid (CI42520; Basic Violet No. 2), Di(4-aminophenyl)(4-amino-3-methylphenyl)carbenium-chlorid (CI42510; Basic Violet No. 14), 1-[(4-Aminophenyl)azo]-7-(trimethylammonio)-2-naphtholchlorid (CI12250; Basic Brown No. 16), 3-[(4-Amino-2,5-dimethoxyphenyl)azo]-N,N,N-trimethylbenzolaminium-chlorid (CI112605, Basic Orange No. 69), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethylammonio)-2-naphthol-chlorid (CI12251; Basic Brown No. 17), 2-((4-Aminophenyl)azo)-1,3-dimethyl-1 H-imidazol-3-ium-chlorid (Basic Orange No. 31), 3,7-Diamino-2,8-dimethyl-5-phenylphenazinium-chlorid (CI50240; Basic Red No. 2), 1,4-Dimethyl-5-[(4-(dimethylamino)phenyl)-azo]-1,2,4-triazolium-chlorid (CI11055; Basic Red No. 22), 1,3-Dimethyl-2-((4-dimethylamino)phenyl)azo-1 H-imidazol-3-ium-chlorid (Basic Red No. 51), 2-Hydroxy-1-[(2-methoxyphenyl)azo]-7-(trimethylammonio)-naphthalin-chlorid (CI12245; Basic Red No. 76), 3-Methyl-1-phenyl-4-[(3-(trimethylammonio)phenyl)azo]-pyrazol-5-on-chlorid (CI12719; Basic Yellow No. 57), 1-Methyl-4-((methyl-phenylhydrazono)methyl)-pyridiniummethylsulfat (Basic Yellow No. 87), 1-(2-Morpholiniumpropylamino)-4-hydroxy-9,10-anthrachinon-methylsulfat, 1-[(3-(Dimethyl-propylaminium)-propyl)amino]-4-(methylamino)-9,10-anthrachinon-chlorid; 1-[Di(2-hydroxyethyl)amino]-3-methyl-4-[(4-nitrophenyl)azo]-benzol (CI11210, Disperse Red No. 17), 1-[Di(2-hydroxyethyl)amino]-4-[(4-nitrophenyl)azo]-benzol, (Disperse Black No. 9), 4-[(4-Aminophenyl)azo]-1-[di(2-hydroxyethyl)amino]-3-methylbenzol, (HC Yellow No. 7), 2,6-Diamino-3-[(pyridin-3-yl)azo]-pyridin und 2-((4-(Ethyl(2-hydroxyethyl)amino)-2-methylphenyl)-azo)-5-nitro-1,3-thiazol (CI111935; Disperse Blue No. 106).

Das erfindungsgemäße Färbemittel (b), das zusätzlich Oxidationsmittel, insbesondere Wasserstoffperoxid oder Wasserstoffperoxidaddukte (z.B.

Natriumpercarbonat oder Harnstoffperoxid) und/oder Persulfate (z.B. Ammoniumpersulfat, Natriumpersulfat oder Kaliumpersulfat) und/oder Perborate, enthält, kann neben den Farbstoffen der allgemeinen Formel (I) zusätzlich noch weitere bekannte, gegenüber Oxidationsmitteln stabile, direktfärbende Farbstoffe enthalten, wie zum Beispiel 3-(2',6'-Diaminopyridyl-3'-azo)-pyridin (= 2,6-Diamino-3-((pyridin-3-yl)azo)pyridin), 2-((4-(Ethyl(2-hydroxyethyl)-amino)-2-methylphenyl)azo-5-nitro-1,3-thiazol (Disperse Blue 106), N,N-Di(2-hydroxyethyl)-3-methyl-4-((4-nitrophenyl)-azo)-anilin (Disperse Red 17, Cl 11210), 3-Diethylamino-7-(4-dimethylaminophenylazo)-5-phenyl-phenaziniumchlorid (CI11050), 4-(2-Thiazolylazo)-resorcin, 4-(((4-Phenylamino)azo)benzosulfonsäure-natriumsalz (Orange IV), 1-((3-Aminopropyl)amino)-9,10-anthracendion (HC Red No. 8), 3',3",4,5,5',5",6,7-Octabromphenolsulfonphtalein (Tetrabromphenol Blue), 1-((4-Amino-3,5-dimethylphenyl)-(2,6-dichlorphenyl)methylen)-3,5-dimethyl-4-imino-2,5-cyclo-hexadien-Phosphorsäure (1:1) (Basic Blue 77), 3',3",5',5"-Tetrabrom-m-kresolsulfonphthalein, 2,4-Dinitro-1-naphthol-7-sulfonsäure-Dinatriumsalz (Acid Yellow 1, Cl 10316), 4-[2'-Hydroxy-1'-naphthyl)azo]-benzosulfonsäure-Natriumsalz (Acid Orange 7, Cl 15510), 3',6'-Dihydroxy-2',4',5',7'-tetraiodospiro-[isobenzo-furan-1(3H), 9'(9H)-xanthen]-3-on-Dinatriumsalz (Acid Red 51, Cl 45430), 6-Hydroxy-5-((2-methoxy-5-methyl-4-sulfophenyl)azo)-2-Naphthalin-sulfonsäure-dinatriumsalz (FD&C Red 40, Cl 16035), 2,4-Dinitro-1-naphthol-Natriumsalz (Acid Yellow 24; Cl 10315), 2',4',5',7'-tetrabrom-4,5,6,7-tetrachlor-3',6'-dihydroxy-Spiro(isobenzofuran-1(3H), 9'[9H]xanthen]-3-on-dinatriumsalz (Acid Red 92; Cl 45410), 4-(2-Hydroxy-1-naphthylazo)-3-methyl-benzolsulfonsäurenatriumsalz (Acid Orange 8, Cl 15575), 2-Amino-1,4-naphthalindion, Dithizon (1,5-Diphenylthiocarbazon), N-(2-Hydroxyethyl))-2-nitro-4-trifluormethyl)anilin (HC Yellow 13), N-(2-hydroxyethyl)-4-nitro-anilin und 4-Chlor-N-(2,3-dihydroxypropyl)-2-nitro-anilin.

Die vorgenannten zusätzlichen direktziehenden Farbstoffe können in dem erfindungsgemäßen Mittel in einer Gesamtmenge von etwa 0,01 bis 4 Gewichtsprozent enthalten sein, wobei der Gesamtgehalt an Farbstoffen in dem erfindungsgemäßen Färbemittel vorzugsweise etwa 0,01 bis 10 Gewichtsprozent, insbesondere 0,1 bis 5 Gewichtsprozent, beträgt.

Das erfindungsgemässe Färbemittel kann weiterhin alle für derartige Zubereitungen bekannten und üblichen Zusatzstoffe, beispielsweise Parfümöle, Komplexbildner, Wachse, Konservierungsstoffe, Verdicker, Alginate, Guar Gum, haarpflegende Substanzen, wie zum Beispiel kationische Polymere oder Lanolinderivate, oder anionische , nichtionische, amphotere oder kationische oberflächenaktive Substanzen enthalten. Vorzugsweise werden amphotere oder nichtionische oberflächenaktive Substanzen, beispielsweise Betaintenside, Propoinate und Glycinate, wie zum Beispiel Cocoamphoglycinate oder Cocoamphdiglacinate, ethoxylierte Tenside mit 1 bis 1000 Ethylenoxid-Einheiten, vorzugsweise mit 1 bis 300 Ethylenoxid-Einheiten, wie zum Beispiel Glyceridalkoxylate, beispielsweise mit 25 Ethylenoxid-Einheiten ethoxyliertes Rizinusöl, Polyglycolamide, ethoxylierte Alkohole und ethoxylierte Fettalkohole (Fettalkoholalkoxylate) und ethoxylierte Fettsäurezuckerester, insbesondere ethoxylierte Sorbitanfettsäureester, eingesetzt. Die vorgenannten Bestandteile werden in den für solche Zwecke üblichen Mengen verwendet, zum Beispiel die oberflächenaktiven Substanzen in einer Konzentration von 0,1 bis 30 Gewichtsprozent, und die Pflegestoffe in einer Menge von 0,1 bis 5 Gewichtsprozent.
Das erfindungsgemässe Färbemittel kann, insbesondere wenn es ein Haarfärbemittel ist, in Form einer wässrigen oder wässrig-alkoholischen Lösung, einer Creme, eines Gels, einer Emulsion oder eines Aerosolschaumes vorliegen, wobei das Haarfärbemittel sowohl in Form eines Einkomponentenpräparates als auch in Form eines Mehrkomponentenpräparates, beispielsweise in Form eines Zweikomponentenpräparates, bei dem das jeweilige Farbstoffderivat der allgemeinen Formel (I) getrennt von den übrigen Bestandteilen abgepackt wird und die Herstellung des gebrauchsfertigen Haarfärbemittels erst unmittelbar vor der Anwendung durch Vermischen der beiden Komponenten erfolgt, konfektioniert sein kann. Das Färbemittel kann, wenn die Farbstoffe zusammen mit einem Oxidationsmittel verwendet werden sollen, auch in Form eines 2-Komponentenpräparates, bei dem die eine Komponente das Oxidationsmittel und die andere Komponente die übrigen Bestandteile des Mittels enthält, konfektioniert sein, wobei das Oxidationsmittel ggfs. ebenfalls aus mehreren Komponenten (z.B. 1. Wasserstoffperoxid und 2. Persulfat) bestehen kann.

Das erfindungsgemäße Färbemittel weist einen pH von etwa 2 bis 10, vorzugsweise etwa 5 bis10, und insbesondere einem neutralen bis basischen pH-Wert von etwa 7 bis 10, auf. Zur Einstellung des erfindungsgemäßen pH-Wertes sind sowohl organische als auch anorganische Säuren oder Basen geeignet. Als geeignete Säuren sind insbesondere α-Hydroxycarbonsäuren, wie zum Beispiel Glycolsäure, Milchsäure, Weinsäure, Zitronensäure oder Äpfelsäure, Ascorbinsäure, Gluconsäurelacton, Essigsäure, Salzsäure oder Phosphorsäure, sowie Mischungen dieser Säure zu nennen. Als geeignete Basen sind inbesonders Natriumcarbonat, Natriumhydrogencarbonat, Alkanolamine, beispielsweise Monoethanolamin oder Triethanolamin, Ammoniak, Aminomethylpropanol und Natriumhydroxid zu nennen.

Die Anwendung des erfindungsgemäßen Färbemittels erfolgt normalerweise indem man eine für die Färbung ausreichende Menge, in der Regel etwa 30 bis 120 Gramm, des Färbemittels (gegebenenfalls unter Zusatz eines geeigneten Oxidationsmittels) auf die Faser aufträgt, das Färbemittel bei etwa 15 bis 45 Grad Celsius etwa 1 bis 60 Minuten, vorzugsweise 5 bis 30 Minuten, einwirken läßt, die Faser sodann gründlich mit Wasser ausspült, gegebenenfalls mit einem Shampoo wäscht und abschließend trocknet.

Das vorstehend beschriebene Färbemittel kann weiterhin, sofern keine Oxidationsmittel der Färbemasse zugesetzt werden, für kosmetische Mittel übliche natürliche oder synthetische Polymere beziehungsweise modifizierte Polymere natürlichen Urprungs enthalten, enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Polyacrylsäure oder Polymethacrylsäure, basische Polymerisate von Estern der Polyacrylsäure, Polymethylacrylsäure und Aminoalkohole, beispielsweise deren Salze oder Quaternisierungsprodukte, Polyacrylnitril, Polyvinylacetate sowie Copolymerisate aus derartigen Verbindungen, wie zum Beispiel Polyvinylpyrrolidon-Vinylacetat, genannt; während als natürliche Polymere oder modifizierte natürliche Polymere beispielsweise Chitosan (entacetyliertes Chitin) oder Chitosanderivate, genannt werden können.

Die vorgenannten Polymere können in dem erfindungsgemäßen Färbemittel in der für solche Mittel üblichen Mengen, insbesondere in einer Menge von etwa 1 bis 5 Gewichtsprozent, enthalten sein. Der pH-Wert des erfindungsgemäßen Tönungsfestigers oder Farbfestigers beträgt vorzugsweise etwa 6 bis 9.

Die Anwendung des Färbemittels mit zusätzlicher Festigung erfolgt in bekannter und üblicher Weise durch Befeuchten des Haares mit dem Festiger, Festlegen (Einlegen) des Haares zur Frisur und anschließende Trocknung.

Das erfindungsgemäße Färbemittel ermöglicht eine hervorragende, gleichmäßige, intensive und äußerst dauerhafte Färbung von Keratinfasern (beispielsweise menschlichen Haaren, Wolle oder Pelzen) ohne nennenswerte Anfärbung der Haut beziehungsweise Kopfhaut, die fünf und mehr Haarwäschen ohne ein merkliches Verblassen der Haarfarbe überdauert.

Die Naphthalinderivate der allgemeinen Formel (I) ermöglichen eine gleichmäßige, intensive und brillante Färbung von Fasern, insbesondere Keratinfasern, wie zum Beispiel menschlichen Haaren, aber auch Wolle oder Pelzen, unter schonenden und hautverträglichen Bedingungen, wobei die Färbungen eine außerordentlich guten Stabilität gegen Licht, Schweiß und Shampoonieren aufweisen. Weiterhin ist bei besonderer Anregung, beispielsweise durch UV-Licht, in bestimmten Fällen eine ausgeprägte Festkörperfluoreszenz der gefärbten Faser zu beobachten.

Die nachfolgenden Beispiele sollen den Gegenstand der Erfindung näher erläutern, ohne diesen hierauf zu beschränken.

### Beispiele

### Beispiel 1: Darstellung von 4-{(E)-[(1,3-Dioxo-2,3-dihydro-1 H-benzo[de]isochinolin-6-yl)imino]methyl}-1-(2-hydroxyethyl)pyridinium-bromid

### Stufe 1: Darstellung von 6-{[(E)-4-Pyridinylmethylidene]amino}-1H-benzo[de]-isochinolin-1,3(2H)-dion

3 g (14,13 mmol) 4-Amino-naphthalin-1,8-dicarbonsäureimid und 7,56 g (70,65 mmol) 4-Pyridincarboxaldehyd werden in 180 ml einer 2:1-Mischung aus konzentrierter Schwefelsäure und Eisessig 2 Stunden lang bei 80 °C gerührt. Anschließend wird der Ansatz auf Eis gegossen und mit NaOH langsam neutralisiert oder schwach alkalisch (ca. pH=8) gestellt.

Der ausgefallene Niederschlag wird abgesaugt und mit viel Wasser gewaschen und im Vakuum getrocknet.
Ausbeute: 2,28 g (54% der Theorie), leuchtend gelbes Pulver
¹H-NMR (d₆-DMSO/300 MHz): δ = 6,46 (s, 1 H), 7,52-7,56 (m, 2H, aromat.), 7,20 (d, J=5,7 Hz, 2H, Pyridyl), 8,38 (m, 1 H, aromat.), 8,65 (d, J= 5,7 Hz, 2H, Pyridyl), 8,71-8,73 (m, 2H, aromat.), 11,41 (s, 1 H, N-H).

### Stufe 2: Darstellung von 4-{(E)-[(1,3-Dioxo-2,3-dihydro-1 H-benzo[de]isochinolin-6-yl)imino]methyl}-1-(2-hydroxvethyl)pyridinium-bromid

Zu einer Lösung von 1,13 g (3,75 mmol) 6-{[(E)-4-Pyridinyimethyliden]-amino}-1 H-benzo[de]isochinolin-1,3(2H)-dion in 60 ml Acetonitril oder Aceton werden 2,34 g (18,75 mmol) Bromethanol getropft und die Lösung anschließend 1 Stunde lang unter Rückfluss erhitzt. Nach dem Einengen auf ca. 1/3 der Lösungsmittelmenge and Abkühlen auf Raumtemperatur wird der entstandene Niederschlag abgesaugt, mit Essigester gewaschen und im Vakuum getrocknet.
Ausbeute: 0,59 g (37% der Theorie), dunkelgelbes Pulver
¹H-NMR (d₆-DMSO/300 MHz): δ = 3,58 (t, J= 13,5 Hz, 2H, Ethyl), 4,01 (t, J= 13,5 Hz, 2H, Ethyl), 6,45 (s, 1 H), 7,54-7,58 (m, 2H, aromat.), 7,19 (d, J=5,8 Hz, 2H, Pyridyl), 8,40 (m, 1 H, aromat.), 8,63 (d, J= 5,8 Hz, 2H, Pyridyl), 8,72-8,75 (m, 2H, aromat.), 11,39 (s, 1 H, N-H).

### Beispiel 2: Darstellung von 3-{2-[Ethyl-4-(6-nitro-1,3-dioxo-1 H-benzo[de]isochinolin-2(3H)-yl)anilino]ethyl}-1-methyl-1H-imidazol-3-ium-bromid

### Stufe 1: Darstellung von 2-{4-[Ethyl(2-hydroxyethyl)amino]phenyl}-6-nitro-1H-benzo[de]isochinolin-1,3(2H)-dion

2,0 g (8,22 mmol) 4-Nitro-naphthalin-1,8-dicarbonsäureanhydrid und 2,68 g N-Ethyl-N-(2-hydroxyethyl)-p-phenylendiaminsulfat-monohydrat (9,04 mmol) werden 1 Stunde lang bei 130 °C in 12 g geschmolzenem Imidazol unter Argonatmosphäre gerührt. Nach dem Abkühlen wird der Ansatz in 400 ml 2 N Salzsäure gegossen, der Niederschlag abgesaugt, mit Wasser gewaschen und getrocknet. Das so erhaltene Produkt wird ohne weitere Reinigung in Stufe 2 weiterverwendet.

### Stufe 2: Darstellung von 2-{4-[(2-Bromoethyl)(ethyl)amino]phenyl}-6-nitro-1H-benzo[de]isochinolin-1,3(2H)-dion

1,5 g (3,7 mmol) 2-{4-[Ethyl(2-hydroxyethyl)amino]phenyl}-6-nitro-1H-benzo[de]iso-chinolin-1,3(2H)-dion werden in 80 ml Chloroform gelöst und zum Sieden erhitzt. Nun werden innerhalb 20 Minuten 10,7 ml (11,1 mmol) Phophortribromid zugetropft und die Lösung noch 2 Stunden unter Rückfluss gekocht. Anschließend wird auf Eis gegossen und die wässrige Phase mit Chloroform extrahiert. Die vereinigten organischen Phasen werden über Magnesiumsulfat getrocknet. Die Reinigung erfolgt mittels Säulenchromatographie an Kieselgel mit Toluol als Laufmittel.
Ausbeute: 0,90 g (52% der Theorie), dunkelrotes. Pulver
¹H-NMR (d₆-DMSO/300 MHz): δ = 1,12 (t, J=8,1 Hz, 3H), 1,86 (q, 2H), 3,58 (t, J=6,9 Hz, 2H), 4,01 (t, J=6,9 Hz, 2H), 6,96 (d, J=4,2 Hz, 2H, Phenyl), 7,83 (d, J=7,6 Hz, 1 H, Naphthalin), 7,95 (d, J=4,4 Hz, 2H, Phenyl), 8,33 (d, J= 8,1 Hz, 1 H, Naphthalin), 8,52 (d, J=8,8 Hz, 1 H, Naphthalin), 8,67 (d, J=8,0 Hz, 1 H, Naphthalin), 9,13 (d, J=8,6 Hz, 1 H, Naphthalin).

### Stufe 3: Darstellung von 3-{2-[Ethyl-4-(6-nitro-1,3-dioxo-1H-benzo[de]iso-chinolin-2(3H)-yl)anilino]ethyl}-1-methyl-1H-imidazol-3-ium bromid

0,5 g (1,07 mmol) 2-{4-[(2-Bromoethyl)(ethyl)amino]phenyl}-6-nitro-1H-benzo[de]iso-chinolin-1,3(2H)-dion werden in 40 ml Acetonitril gelöst. Nach Zugabe von 0,44 g (5,35 mmol) N-Methyl-imidazol wird der Ansatz 2 Stunden lang unter Rückfluss gerührt. Nach Entfernung des Lösungsmittels im Vakkuum wird der Niederschlag abfiltriert, mit Essigester gewaschen und getrocknet.
Ausbeute: 0,32 g (54% der Theorie), rotbraunes Pulver
UV/Vis DMSO): λₘₐₓ = 409, 520 nm.

### Beispiel 3: Darstellung von 2-[(3-Methyl-1,3-benzothiazol-2(3H)-ylidene)amino]-6-nitro-1 H-benzo[de]isochinolin-1,3(2H)-dion

Durch Kondensation (Synthese und Aufarbeitung in Analogie zu Beispiel 2, Stufe 1) von 2,3-Dihydro-3-methyl-2-benzothiazolon-hydrazonhydrochlorid-hydrat mit 4-Nitro-naphthalin-1,8-dicarbonsäureanhydrid in Imidazol wird das Produkt als gelbes Pulver erhalten. Die Reinigung erfolgt mittels Säulenchromatographie an Kieselgel.
UV/Vis (CHCl₃): λₘₐₓ = 301, 345 nm.

### Beispiel 4: Darstellung von 2-(1,3-Benzothiazol-2-yl)-6-nitro-1 H-benzo[de]iso-chinolin-1,3(2H)-dion

Analog zu Beispiel 3 wird aus 2-Amino-benzothiazol und 4-Nitro-naphthalin-1,8-dicarbonsäureanhydrid in Imidazol das Produkt als orangegelbes Pulver isoliert. Die Reinigung erfolgt mittels
Säulenchromatographie an Kieselgel.
UV/Vis (CHCl₃): λₘₐₓ = 345, 379 nm.

### Beispiel 5: Darstellung von 2-{2-[(2-hydroxyethyl)amino]-4-methoxyphenyl}-5-nitro-1 H-benzo[de]isochinolin-1,3(2H)-dion

Die Reaktion von N-(2-Hydroxyethyl)-5-methoxy-2-amino-Anilin mit 3-Nitro-naphthalin-1,8-dicarbonsäureanhydrid in Imidazol (Synthese und Aufarbeitung analog Beispiel 2, Stufe1) liefert das Produkt als dunkelrotes Pulver. Zur Reinigung wird das Rohprodukt über Kieselgel in einer
Ethanol/Aceton-Mischung filtriert.
UV/Vis (CHCl₃): λₘₐₓ = 411, 524 nm.

### Beispiel 6: Haarfärbemittel (ohne Oxidationsmittel)

| | |
|---|---|
| 2,5 mmol | Farbstoff der allgemeinen Formel (I) |
| 5,0 g | Ethanol |
| 4,0 g | Decylpolyglucose |
| 0,2 g | Ethylendiaminotetraessigsäure-Dinatriumsalz-Hydrat |
| ad 100,0 g | Wasser, vollentsalzt |

Die Färbelösung wird durch Zugabe von Ammoniak auf einen pH-Wert von 7 bis 10 eingestellt.

Die Haarfärbung erfolgt, indem eine für die Haarfärbung ausreichende Menge des Färbemittels auf das Haar aufgetragen wird, nach einer Einwirkzeit von 30 Minuten bei 40 °C das Haar mit lauwarmen Wasser ausgespült und getrocknet wird. Die Färbeergebnisse sind in der nachfolgenden Tabelle 1 zusammengefasst.

### Beispiel 7: Haarfärbemittel (mit Oxidationsmittel)

| | |
|---|---|
| 0,100 g | Farbstoff der allgemeinen Formel (I) |
| 1,000 g | Kaliumpersulfat |
| 1,500 g | Ammoniumpersulfat |
| 1,200 g | Natriumsilicat |
| 0,625 g | Magnesiumoxid |
| 0,250 g | Hydroxyethylcellulose |
| 0,300 g | Seifenperlen |
| 0,100 g | disperse Kieselsäure |
| 0,025 g | Dinatrium-EDTA |
| 10,000 g | Wasserstoffperoxid (12% in Wasser) |

Die angegebenen Komponenten werden zu einer homogenen Masse vermischt, so dass keine Farbstoffpatikel mehr zu erkennen sind. Dann wird eine für die Haarfärbung ausreichende Menge der vorgenannten Färbemasse auf das Haar aufgetragen. Nach einer Einwirkzeit von 45 Minuten bei 40 °C wird das Haar mit lauwarmen Wasser ausgespült und mit einem sauren Conditioner behandelt, erneut ausgespült und sodann getrocknet.

Die Färbeergebnisse sind in der nachfolgenden Tabelle 1 zusammengefasst.

**Tabelle 1**

| **Beispiel- Nr.** | **Farbstoff der allgemeinen Formel (I)** | **Färbung** |
|---|---|---|
| **6** | 3-{2-[Ethyl-4-(6-nitro-1,3-dioxo-1H-benzo[de]isochinolin-2(3H)-yl)anilino]-ethyl}-1-methyl-1H-imidazol-3-ium-bromid | rot-orange |
| **7** | 4-{(E)-[(1,3-Dioxo-2,3-dihydro-1H-benzo- [de]isochinolin-6-yl)imino]-methyl}-1-(2-hydroxyethyl)pyridinium-bromid | leuchtend gelb; gelbgrüne Fluoreszenz bei UV- Anregung |

### Beispiel 8: Haarfärbemittel (mit Oxidationsmittel und weiterem Direktfarbstoff)

| | |
|---|---|
| 0,100 g | 4-{(E)-[(1,3-Dioxo-2,3-dihydro-1H-benzo-[de]isochinolin-6-yl)imino]-methyl}-1-(2-hydroxyethyl)pyridinium-bromid |
| 0,050 g | 3',3",4,5,5',5",6,7-Octabromphenolsulfonphtalein (Tetrabromphenol Blue) |
| 1,000 g | Kaliumpersulfat |
| 1,500 g | Ammoniumpersulfat |
| 1,200 g | Natriumsilicat |
| 0,625 g | Magnesiumoxid |
| 0,250 g | Hydroxyethylcellulose |
| 0,300 g | Seifenperlen |
| 0,100 g | disperse Kieselsäure |
| 0,025 g | Dinatrium-EDTA |
| 10,000 g | Wasserstoffperoxid (12% in Wasser) |

Die angegebenen Komponenten werden zu einer homogenen Masse vermischt, so dass keine Farbstoffpatikel mehr zu erkennen sind. Dann wird eine für die Haarfärbung ausreichende Menge der vorgenannten Färbemasse auf das Haar aufgetragen. Nach einer Einwirkzeit von 45 Minuten bei 40 °C wird das Haar mit lauwarmen Wasser ausgespült und anschließend getrocknet. Es wird eine hellblaue Färbung erhalten.

### Beispiel 9: Haarfärbemittel (mit Oxidationsmittel und weiterem Direktfarbstoff)

| | |
|---|---|
| 0,100 g | 4-{(E)-[(1,3-Dioxo-2,3-dihydro-1 H-benzo-[de]isochinolin-6-yl)imino]-methyl}-1-(2-hydroxyethyl)pyridinium-bromid |
| 0,050 g | 2',4',5',7'-Tetrabrom-4,5,6,7-tetrachlor-3',6'-dihydroxy-spiro(isobenzofuran-1(3H), 9'[9H]xanthen]-3-on-dinatriumsalz (Acid Red 92; Cl 45410) |
| 1,000 g | Kaliumpersulfat |
| 1,500 g | Ammoniumpersulfat |
| 1,200 g | Natriumsilicat |
| 0,625 g | Magnesiumoxid |
| 0,250 g | Hydroxyethylcellulose |
| 0,300 g | Seifenperlen |
| 0,100 g | disperse Kieselsäure |
| 0,025 g | Dinatrium-EDTA |
| 10,000 g | Wasserstoffperoxid (12% in Wasser) |

Die angegebenen Komponenten werden zu einer homogenen Masse vermischt, so dass keine Farbstoffpatikel mehr zu erkennen sind. Dann wird eine für die Haarfärbung ausreichende Menge der vorgenannten Färbemasse auf das Haar aufgetragen. Nach einer Einwirkzeit von 45 Minuten bei 40 °C wird das Haar mit lauwarmen Wasser ausgespült und anschließend getrocknet. Es wird eine rosarote Färbung erhalten.

Alle in der vorliegenden Anmeldung genannten Prozentangaben stellen soweit nicht anders angegeben Gewichtsprozente dar.

## Patentansprüche

1. Mittel zum Färben von Keratinfasern, **dadurch gekennzeichnet, dass** es mindestens ein Naphthalinderivat der allgemeinen Formel (1) enthält, 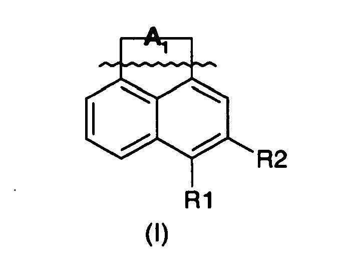 worin
**A₁** eine Teilstruktur der Formeln (II), (III), (IV), (V) und (VI) darstellt, 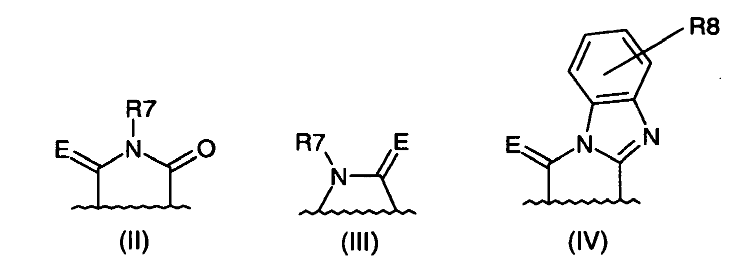 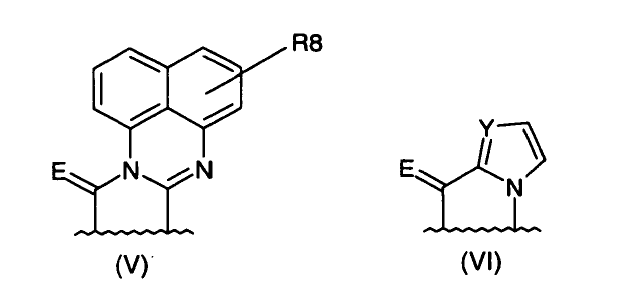 darstellt, worin
**E** ein Sauerstoff oder ein Schwefelatom darstellt;
**Y** für ein Stickstoffatom oder ein quaternäres Stickstoffatom steht, welches mit verzweigten oder linearen C₁-C₆-Alkylgruppen, verzweigten oder linearen C₂-C₄-Hydroxyalkylgruppen oder verzweigten oder linearen C₄-C₆-Polyhydroxyalkylgruppen substituiert ist;
**R₁** und **R₂** unabhängig voneinander für Wasserstoff, eine -N-R₃R₄-Gruppe, eine Nitrogruppe, eine -N=N-R₅-Gruppe oder eine -N=C-R₃R₆-Gruppe stehen;
**R₃** und **R₄** gleich oder verschieden sein können und Wasserstoff, eine C₁-C₆-Alkylaminogruppe, eine C₁-C₆-N,N-Dialkylaminogruppe, eine C₁-C₆-Alkylcyanogruppe, eine Methoxymethylgruppe, eine tert-Butylgruppe, eine Isopropylgruppe, eine C₁-C₆-Alkylgruppe, eine C₁-C₆-Alkyloxygruppe, eine C₁-C₆-Hydroxyalkylgruppe, eine C₁-C₆-Alkylcarbonsäuregruppe, eine C₁-C₆-Alkylcarbonsäureestergruppe, eine C₁-C₆-Alkylcarbonsäureamidgruppe, eine C₁-C₆-Alkylsulfonsäuregruppe, eine C₁-C₆-Alkylsulfonsäureestergruppe, eine C₁-C₆-Alkylsulfonsäureamidgruppe, eine Phenylgruppe oder eine -(L)-B⁺-Gruppe darstellen;
**R₅** eine Gruppe der Formel (VII) oder (VIII) darstellt; 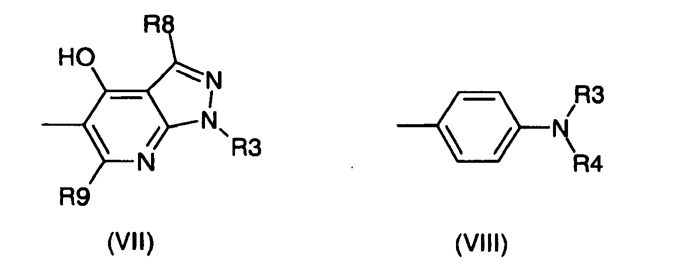
**R₆** eine Verbindung der Formel (IX), (X) oder (XI) darstellt; 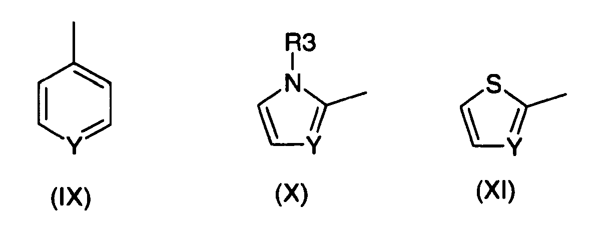
**R₇** ein Wasserstoffatom, einen aliphatischen C₁-C₆-Alkylrest, der linear oder verzweigt, unsubstituiert oder mit einer oder mehreren Hydroxylgruppen oder kationischen Resten des Typs B⁺ substituiert ist; einen aromatischen Rest der allgemeinen Formel (XII) oder (XIII); 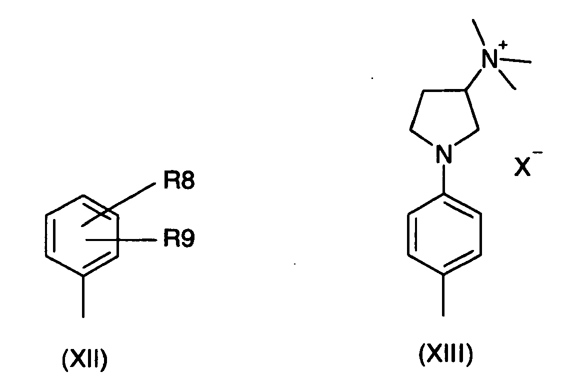 oder einen fünfgliedrigen oder sechsgliedrigen Heterozyklus oder heterozyklische Bizyklus der allgemeinen Formeln (XIV), (XV), (XVI) oder (XVII) darstellt; 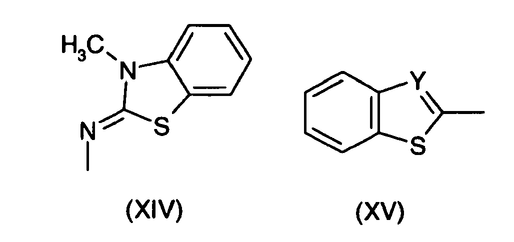 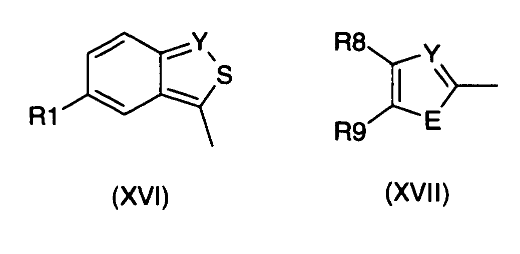
**R₈** und **R₉** gleich oder verschieden sein können und Wasserstoff, eine Aminogruppe, eine C₁-C₆-Alkylaminogruppe, eine C₁-C₆-N,N-Dialkylaminogruppe, eine C₁-C₆-N,N-(Dihydroxyalkyl)aminogruppe, Fluor, Chlor, Brom, Jod, eine Cyanogruppe, eine C₁-C₆-Alkylcyanogruppe, eine Methoxymethylgruppe, eine tert-Butylgruppe, eine Isopropylgruppe, eine C₁-C₆-Alkylgruppe, eine C₁-C₆-Alkyloxygruppe, eine C₁-C₆-Hydroxyalkylgruppe, eine C₁-C₆-Hydroxyalkyloxygruppe, eine C₁-C₆-Alkylcarbonsäuregruppe, eine C₁-C₆-Alkylcarbonsäureestergruppe, eine C₁-C₆-Alkylcarbonsäureamidgruppe, eine C₁-C₆-Alkylsulfonsäuregruppe, eine C₁-C₆-Alkylsulfonsäureestergruppe, eine C₁-C₆-Alkylsulfonsäureamidgruppe, eine Phenylgruppe, eine Sulfonsäuregruppe oder eine -(L)-B⁺ -Gruppe darstellen;
**L** für eine C₁-C₆-Alkylengruppe steht;
**B⁺** für eine aromatische heterozyklische quaternäre Ammoniumverbindung; eine nicht-aromatische heterozyklische quaternäre Ammoniumverbindung; eine quaternäre Alkylammoniumverbindung oder Arylammoniumverbindung der Formel NRₐR_{b}R_{c}, wobei **Rₐ, R_{b},** und **R_{c}** unabhängig voneinander einen Benzylrest, einen Phenylrest oder einen C₁-bis C₆-Alkylrest bedeuten, wobei die vorgenannten Alkylreste unsubstituiert oder mit einer oder mehreren Hydroxylgruppen oder Aminogruppen substituiert sein können; oder
eine quaternäre Phosphoniumgruppe steht; und **X⁻** ein Anion ist.

2. Mittel nach Anspruch 1, **dadurch gekennzeichnet, dass** das Naphthalinderivat der allgemeinen Formel (I) ausgewählt ist aus 3-((E)-{4-[Ethyl(2-hydroxyethyl)amino]phenyl}diazenyl)-8-methyl-7-oxo-7H-benzo[de]-imidazo-[1,2-a]chinolin-8-ium-methylsulfat, 3-{(E)-[4-Hydroxy-1-(2-hydroxyethyl)-3,6-dimethyl-1H-pyrazolo[3,4-b]pyridin-5-yl]diazenyl}-8-methyl-7-oxo-7H-benzo[de]imidazo[1,2-a]chinolin-8-ium-methylsulfat, 3-Methyl-2-(5-nitro-2-oxobenzo[cd]indol-1 (2H)-yl)-1,3-benzothiazol-3-ium-methylsulfat, 3-Nitro-7H-benzo[de]imidazo[1,2-a]chinolin-7-on, 2-(5-(Dimethylamino)-2-oxobenzo[cd]indol-1(2H)-yl)-3-methyl-1,3-benzothiazol-3-ium-methylsulfat, 3-{2-[4-(5-(Dimethylamino)-1,3-dioxo-1H-benzo[de]isochinolin-2(3H)-yl)(ethyl)anilino]ethyl}-1-methyl-1H-imidazol-3-ium-bromid, 5-((E)-{1-[4-(Dimethylamino)phenyl]-2-oxo-1,2-dihydrobenzo-[cd]indol-6-yl}diazenyl)-4-hydroxy-1-(2-hydroxyethyl)-3,6,7-trimethyl-1 H-pyrazolo[3,4-b]pyridin-7-ium-methylsulfat, 3-(Dimethylamino)-8-methyl-7-oxo-7H-benzo[de]imidazo[1,2-a]chinolin-8-ium-methylsulfat, 1-{4-[bis(Hydroxyethyl)amino]phenyl}-6-nitrobenzo[cd]indol-2(1H)-on, 3-{2-[Ethyl-4-(6-nitro-2-oxobenzo[cd]indol-1 (2H)-yl)anilino]ethyl}-1-methyl-1 H-imidazol-3-ium-bromid, 4-((E)-{[2-(3,4-Dimethoxyphenyl)-1,3-dioxo-2,3-dihydro-1 H-benzo[de]isoquinolin-6-yl]imino}methyl)-1-(2-hydroxyethyl)-pyridinium-bromid, 2-(3,4-Dimethoxyphenyl)-6-nitro-1 H-benzo[de]-isochinolin-1,3(2H)-dion, 1-(3,4-Dimethoxyphenyl)-6-nitrobenzo-[cd]indol-2(1 H)-on, 1-Methyl-3-[2-({2-[((2Z)-3-methyl-1,3-benzthiazol-2(3H)-ylidene)amino]-1,3-dioxo-2,3-dihydro-1 H-benzo[de]isochinolin-6-yl}amino)ethyl]-1 H-imidazol-3-ium-bromid, 1-(2-Hydroxyethyl)-4-{(E)-[(7-oxo-7H-benzimidazo[2,1-a]benzo[de]isochuinolin-2-yl)imino]methyl}-pyridinium-bromid, 1-(2-Hydroxyethyl-4-{(E)-[7-thioxo-7H-benzimidazo[2,1-a]benzo[de]isochinolin-2-yl)imino]methyl}-pyridinium-bromid, 2-{Ethyl-4-[(E)-(14-oxo-14H-benzo[4,5]isochinolino[2,1-a]perimidin-9-yl)diazenyl]-anilino}-N,N,N-trimethylethylethanaminium-bromid, 2-{Ethyl-4-[(E)-(7-oxo-7H-benzimidazo[2,1-a]benzo[de]isochinolin-2-yl)diazenyl]anilino}-N,N,N-trimethylethanaminium-bromid, 2-{Ethyl-4-[(E)-(7-thioxo-7H-benzimidazo-[2,1-a]benzo[de]isochinolin-2-yl)diazenyl]anilino}-N,N,N-trimethylethanaminium-bromid, 2-[4-[(E)-(1,3-dioxo-2,3-dihydro-1H-benzo[de]isochinolin-6-yl)diazenyl](ethyl)anilino]-N,N,N-trimethylethanaminium-bromid, 1-(2-Hydroxyethyl)-4-[(E)-({2-[((2Z)-3-methyl-1,3-benzothiazol-2(3H)-ylidene)amino]-1,3-dioxo-2,3-dihydro-1 H-benzo[de]isochinolin-5-yl}imino)-methyl]pyridinium-bromid, 4-{(E)-[(1,3-Dioxo-2,3-dihydro-1 H-benzo[de]-isochinolin-6-yl)imino]methyl}-1-(2-hydroxyethyl)pyridinium-bromid, 2-[4-((E)-{1-[4-(Dimethylamino)phenyl]-2-oxo-1,2-dihydrobenzo[cd]indol-5-yl}diazenyl)(ethyl)anilino]-N,N,N-trimethylethanaminium-bromid, 3-{2-[Ethyl-4-(2-oxo-5-((E)-{3,4,6-trimethyl-1-[2-(1-methyl-1H-imidazol-3-ium-3-yl)ethyl-1H-pyrazolo[3,4-b]pyridin-5-yl}diazenyl)benzo[cd]indol-1(2H)-yl)anilino]ethyl}-1-methyl-1H-imidazol-3-ium-dibromid, 1-(2-Hydroxyethyl)-4-[(E)-({2-[((2Z)-3-methyl-1,3-benzothiazol-2(3H)-ylidene)amino]-1,3-dioxo-2,3-dihydro-1H-benzo[de]isochinolin-6-yl}imino)methyl]pyridinium-bromid, 2-(6-(Dimethylamino)-1,3-dioxo-1 H-benzo[de]isoquinolin-2(3H)-yl)-3,4,5-trimethyl-1,3-thiazol-3-ium-methylsulfat, 2-(5-(Dimethylamino)-2-oxobenzo[cd]indol-1 (2H)-yl)-3,4,5-trimethyl-1,3-thiazol-3-ium-methylsulfat, 3-{2-[Ethyl-4-(6-nitro-1,3-dioxo-1H-benzo[de]isochinolin-2(3H)-yl)anilino]ethyl}-1-methyl-1H-imidazol-3-ium-bromid, 3-{2-[5-Methoxy-2-(6-nitro-1,3-dioxo-1H-benzo[de]isochinolin-2(3H)-yl)anilino]ethyl}-1-methyl-1H-imidazol-3-ium-bromid, 2-[(3-methyl-1,3-benzothiazol-2(3H)-ylidene)amino]-6-nitro-1H-benzo[de]isochinolin-1,3(2H)-dion, 2-(1,3-Benzothiazol-2-yl)-6-nitro-1H-benzo[de]isochinolin-1,3(2H)-dion, 1-(2-Hydroxyethyl)-4-{(E)-[(14-oxo-14H-benzo[4,5]isochino-[2,1-a]perimidin-9-yl)imino]methyl}pyridinium-bromid und 2-{2-[(2-Hydroxyethyl)amino]-4-methoxyphenyl}-5-nitro-1H-benzo[de]isochinolin-1,3(2H)-dion.

3. Mittel nach Anspruch 1 oder 2, **dadurch gekennzeichnet, dass** dass das Naphthalinderivat der allgemeinen Formel (I) ausgewählt ist aus 3-((E)-{4-[Ethyl(2-hydroxyethyl)amino]phenyl}diazenyl)-8-methyl-7-oxo-7H-benzo[de]-imidazo-[1,2-a]chinolin-8-ium-methylsulfat, 3-{(E)-[4-Hydroxy-1-(2-hydroxyethyl)-3,6-dimethyl-1 H-pyrazolo[3,4-b]pyridin-5-yl]diazenyl}-8-methyl-7-oxo-7H-benzo[de]imidazo[1,2-a]quinolin-8-ium-methylsulfat, 3-Methyl-2-(5-nitro-2-oxobenzo[cd]indol-1 (2H)-yl)-1,3-benzothiazol-3-ium-methylsulfat, 2-(5-(Dimethylamino)-2-oxobenzo[cd]indol-1 (2H)-yl)-3-methyl-1,3-benzothiazol-3-ium-methylsulfat, 5-((E)-{1-[4-(Dimethylamino)-phenyl]-2-oxo-1,2-dihydrobenzo[cd]indol-6-yl}diazenyl)-4-hydroxy-1-(2-hydroxyethyl)-3,6,7-trimethyl-1H-pyrazolo[3,4-b]pyridin-7-ium-methylsulfat, 1-{4-[bis(Hydroxyethyl)amino]phenyl}-6-nitrobenzo[cd]indol-2(1H)-on, 3-{2-[Ethyl-4-(6-nitro-2-oxobenzo[cd]indol-1 (2H)-yl)anilino]ethyl}-1-methyl-1H-imidazol-3-ium-bromid, 1-(2-Hydroxyethyl)-4-{(E)-[(7-oxo-7H-benzimidazo[2,1-a]benzo[de]isochinolin-2-yl)imino]methyl}pyridinium-bromid, 4-{(E)-[(1,3-Dioxo-2,3-dihydro-1H-benzo[de]isochinolin-6-yl)imino]methyl}-1-(2-hydroxyethyl)pyridinium-bromid, 2-[4-((E)-{1-[4-(Dimethylamino)phenyl]-2-oxo-1,2-dihydrobenzo[cd]indol-5-yl}diazenyl)-(ethyl)anilino]-N,N,N-trimethylethanaminium-bromid, 2-[(3-Methyl-1,3-benzothiazol-2(3H)-ylidene)amino]-6-nitro-1 H-benzo[de]isochinolin-1,3(2H)-dion, 2-(1,3-Benzothiazol-2-yl)-6-nitro-1H-benzo[de]isochinolin-1,3(2H)-dion, 1-(2-Hydroxyethyl)-4-{(E)-[(14-oxo-14H-benzo[4,5]isoquino-[2,1-a]perimidin-9-yl)imino]methyl}pyridinium-bromid und 2-{2-[(2-Hydroxyethyl)amino]-4-methoxyphenyl}-5-nitro-1H-benzo[de]isochinolin-1,3(2H)-dion.

4. Mittel nach einem der Ansprüche 1 bis 3, **dadurch gekennzeichnet, dass** es zusätzlich mindestens ein Oxidationsmittel enthält.

5. Mittel nach Anspruch 4, **dadurch gekennzeichnet, dass** das Oxidationsmittel ausgewählt ist aus Wasserstoffperoxid oder Wasserstoffperoxidaddukten, Persulfaten und Perboraten.

6. Mittel nach einem der Ansprüche 1 bis 3, **dadurch gekennzeichnet, dass** es mindestens ein für kosmetische Mittel übliches natürliches oder synthetisches Polymer beziehungsweise modifiziertes Polymer natürlichen Urprungs enthält.

7. Mittel nach Anspruch 6, **dadurch gekennzeichnet, dass** es das Polymer in einer Menge von 1 bis 5 Gerwichtsprozent enthält.

8. Mittel nach einem der Ansprüche 1 bis 7, **dadurch gekennzeichnet, dass** es das Naphthalinderivat der allgemeinen Formel (1) in einer Menge von 0,01 bis 10 Gewichtsprozent enthält.

9. Mittel nach einem der Ansprüche 1 bis 8, **dadurch gekennzeichnet, dass** es ein Haarfärbemittel ist.

10. Naphthalinderivat **dadurch gekennzeichnet, dass** es ausgewählt ist aus 3-((E)-{4-[Ethyl(2-hydroxyethyl)amino]phenyl}diazenyl)-8-methyl-7-oxo-7H-benzo[de]-imidazo-[1,2-a]chinolin-8-ium-methylsulfat, 3-{(E)-[4-Hydroxy-1-(2-hydroxyethyl)-3,6-dimethyl-1 H-pyrazolo[3,4-b]pyridin-5-yl]diazenyl}-8-methyl-7-oxo-7H-benzo[de]imidazo[1,2-a]chinolin-8-ium-methylsulfat, 3-Methyl-2-(5-nitro-2-oxobenzo[cd]indol-1(2H)-yl)-1,3-benzothiazol-3-ium- methylsulfat, 3-Nitro-7H-benzo[de]imidazo[1,2-a]chinolin-7-on, 2-(5-(Dimethylamino)-2-oxobenzo[cd]indol-1 (2H)-yl)-3-methyl-1,3-benzothiazol-3-ium-methylsulfat, 3-{2-[4-(5-(Dimethylamino)-1,3-dioxo-1H-benzo[de]isochinolin-2(3H)-yl)(ethyl)anilino]ethyl}-1-methyl-1 H-imidazol-3-ium-bromid, 5-((E)-{1-[4-(Dimethylamino)phenyl]-2-oxo-1,2-dihydrobenzo-[cd]indol-6-yl}diazenyl)-4-hydroxy-1-(2-hydroxyethyl)-3,6,7-trimethyl-1 H-pyrazolo[3,4-b]pyridin-7-ium-methylsulfat, 3-(Dimethylamino)-8-methyl-7-oxo-7H-benzo[de]imidazo[1,2-a]chinolin-8-ium-methylsulfat, 1-{4-[bis(Hydroxyethyl)amino]phenyl}-6-nitrobenzo[cd]indol-2(1H)-on, 3-{2-[Ethyl-4-(6-nitro-2-oxobenzo[cd]indol-1 (2H)-yl)anilino]ethyl}-1-methyl-1 H-imidazol-3-ium-bromid, 4-((E)-{[2-(3,4-Dimethoxyphenyl)-1,3-dioxo-2,3-dihydro-1H-benzo[de]isoquinolin-6-yl]imino}methyl)-1-(2-hydroxyethyl)-pyridinium-bromid, 2-(3,4-Dimethoxyphenyl)-6-nitro-1H-benzo[de]-isochinolin-1,3(2H)-dion, 1-(3,4-Dimethoxyphenyl)-6-nitrobenzo-[cd]indol-2(1H)-on, 1-Methyl-3-[2-({2-[((2Z)-3-methyl-1,3-benzthiazol-2(3H)-ylidene)amino]-1,3-dioxo-2,3-dihydro-1H-benzo[de]isochinolin-6-yl}amino)ethyl]-1H-imidazol-3-ium-bromid, 1-(2-Hydroxyethyl)-4-{(E)-[(7-oxo-7H-benzimidazo[2,1-a]benzo[de]isochuinolin-2-yl)imino]methyl}-pyridinium-bromid, 1-(2-Hydroxyethyl-4-{(E)-[7-thioxo-7H-benzimidazo[2,1-a]benzo[de]isochinolin-2-yl)imino]methyl}-pyridinium-bromid, 2-{Ethyl-4-[(E)-(14-oxo-14H-benzo[4,5]isochinolino[2,1-a]perimidin-9-yl)diazenyl]-anilino}-N,N,N-trimethylethylethanaminium-bromid, 2-{Ethyl-4-[(E)-(7-oxo-7H-benzimidazo[2,1-a]benzo[de]isochinolin-2-yl)diazenyl]anilino}-N,N,N-trimethylethanaminium-bromid, 2-{Ethyl-4-[(E)-(7-thioxo-7H-benzimidazo-[2,1-a]benzo[de]isochinolin-2-yl)diazenyl]anilino}-N,N,N-trimethylethanaminium-bromid, 2-[4-[(E)-(1,3-dioxo-2,3-dihydro-1 H-benzo[de]isochinolin-6-yl)diazenyl](ethyl)anilino]-N,N,N-trimethylethanaminium-bromid, 1-(2-Hydroxyethyl)-4-[(E)-({2-[((2Z)-3-methyl-1,3-benzothiazol-2(3H)-ylidene)amino]-1,3-dioxo-2,3-dihydro-1 H-benzo[de]isochinolin-5-yl}imino)-methyl]pyridinium-bromid, 4-{(E)-[(1,3-Dioxo-2,3-dihydro-1 H-benzo[de]-isochinolin-6-yl)imino]methyl}-1-(2-hydroxyethyl)pyridinium-bromid, 2-[4-((E)-{1-[4-(Dimethylamino)phenyl]-2-oxo-1,2-dihydrobenzo[cd]indol-5-yl}diazenyl)(ethyl)anilino]-N,N,N-trimethylethanaminium-bromid, 3-{2-[Ethyl-4-(2-oxo-5-((E)-{3,4,6-trimethyl-1-[2-(1-methyl-1H-imidazol-3-ium-3-yl)ethyl-1H-pyrazolo[3,4-b]pyridin-5-yl}diazenyl)benzo[cd]indol-1(2H)-yl)anilino]ethyl}-1-methyl-1 H-imidazol-3-ium-dibromid, 1-(2-Hydroxyethyl)-4-[(E)-({2-[((2Z)-3-methyl-1,3-benzothiazol-2(3H)-ylidene)amino]-1,3-dioxo-2,3-dihydro-1 H-benzo[de]isochinolin-6-yl}imino)methyl]pyridinium-bromid, 2-(6-(Dimethylamino)-1,3-dioxo-1 H-benzo[de]isoquinolin-2(3H)-yl)-3,4,5-trimethyl-1,3-thiazol-3-ium-methylsulfat, 2-(5-(Dimethylamino)-2-oxobenio[cd]indol-1 (2H)-yl)-3,4,5-trimethyl-1,3-thiazol-3-ium-methylsulfat, 3-{2-[Ethyl-4-(6-nitro-1,3-dioxo-1H-benzo[de]isochinolin-2(3H)-yl)anilino]ethyl}-1-methyl-1H-imidazol-3-ium-bromid, 3-{2-[5-Methoxy-2-(6-nitro-1,3-dioxo-1H-benzo[de]isochinolin-2(3H)-yl)anilino]ethyl}-1-methyl-1H-imidazol-3-ium-bromid, 2-[(3-methyl-1,3-benzothiazol-2(3H)-ylidene)amino]-6-nitro-1H-benzo[de]isochinolin-1,3(2H)-dion, 2-(1,3-Benzothiazol-2-yl)-6-nitro-1H-benzo[de]isochinolin-1,3(2H)-dion, 1-(2-Hydroxyethyl)-4-{(E)-[(14-oxo-14H-benzo[4,5]isochino-[2,1-a]perimidin-9-yl)imino]methyl}pyridinium-bromid und 2-{2-[(2-Hydroxyethyl)amino]-4-methoxyphenyl}-5-nitro-1H-benzo[de]isochinolin-1,3(2H)-dion.

11. Naphthalinderivat nach Anspruch 10, **dadurch gekennzeichnet, dass** es ausgewählt ist aus 3-((E)-{4-[Ethyl(2-hydroxyethyl)amino]-phenyl}diazenyl)-8-methyl-7-oxo-7H-benzo[de]-imidazo-[1,2-a]chinolin-8-ium-methylsulfat, 3-{(E)-[4-Hydroxy-1-(2-hydroxyethyl)-3,6-dimethyl-1 H-pyrazolo[3,4-b]pyridin-5-yl]diazenyl}-8-methyl-7-oxo-7H-benzo[de]imidazo[1,2-a]quinolin-8-ium-methylsulfat, 3-Methyl-2-(5-nitro-2-oxobenzo[cd]indol-1 (2H)-yl)-1,3-benzothiazol-3-ium-methylsulfat, 2-(5-(Dimethylamino)-2-oxobenzo[cd]indol-1 (2H)-yl)-3-methyl-1,3-benzothiazol-3-ium-methylsulfat, 5-((E)-{1-[4-(Dimethylamino)-phenyl]-2-oxo-1,2-dihydrobenzo[cd]indol-6-yl}diazenyl)-4-hydroxy-1-(2-hydroxyethyl)-3,6,7-trimethyl-1 H-pyrazolo[3,4-b]pyridin-7-ium-methylsulfat, 1-{4-[bis(Hydroxyethyl)amino]phenyl}-6-nitrobenzo[cd]indol-2(1H)-on, 3-{2-[Ethyl-4-(6-nitro-2-oxobenzo[cd]indol-1(2H)-yl)anilino]ethyl}-1-methyl-1H-imidazol-3-ium-bromid, 1-(2-Hydroxyethyl)-4-{(E)-[(7-oxo-7H-benzimidazo[2,1-a]benzo[de]isochinolin-2-yl)imino]methyl}pyridinium-bromid, 4-{(E)-[(1,3-Dioxo-2,3-dihydro-1H-benzo[de]isochinolin-6-yl)imino]methyl}-1 -(2-hydroxyethyl)pyridinium-bromid, 2-[4-((E)-{1-[4-(Dimethylamino)phenyl]-2-oxo-1,2-dihydrobenzo[cd]indol-5-yl}diazenyl)-(ethyl)anilino]-N,N,N-trimethylethanaminium-bromid, 2-[(3-Methyl-1,3-benzothiazol-2(3H)-ylidene)amino]-6-nitro-1 H-benzo[de]isochinolin-1,3(2H)-dion, 2-(1,3-Benzothiazol-2-yl)-6-nitro-1 H-benzo[de]isochinolin-1,3(2H)-dion, 1-(2-Hydroxyethyl)-4-{(E)-[(14-oxo-14H-benzo[4,5]isoquino-[2,1-a]perimidin-9-yl)imino]methyl}pyridinium-bromid und 2-{2-[(2-Hydroxyethyl)amino]-4-methoxyphenyl}-5-nitro-1H-benzo[de]isochinolin-1,3(2H)-dion.

## Claims

1. Agent for dyeing keratin fibres, **characterized in that** it comprises at least one naphthalene derivative of the general formula (I), 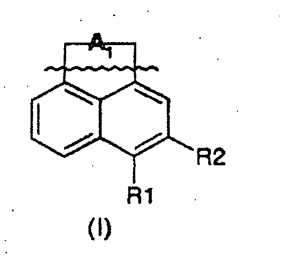 in which
**A₁** is a part structure of the formulae (II), (III), (IV) , (V) and (VI) , 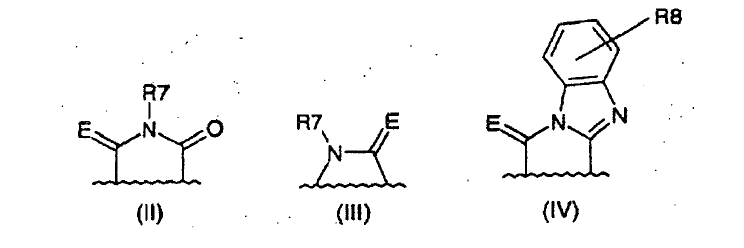 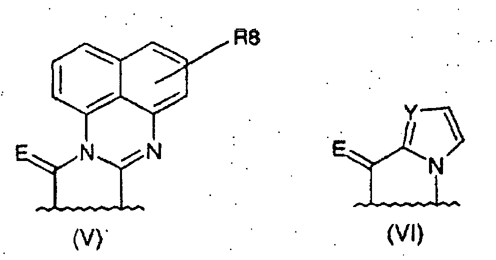 in which
**E** is an oxygen or a sulphur atom;
**Y** is a nitrogen atom or a quaternary nitrogen atom which is substituted by branched or linear C₁-C₆-alkyl groups, branched or linear C₂-C₄-hydroxyalkyl groups or branched or linear C₄-C₆-polyhydroxyalkyl groups;
**R₁** and **R₂,** independently of one another, are hydrogen, a -N-R₃R₄ group, a nitro group, an -N=N-R₅ group or a -N=C-R₃R₆ group;
**R₃** and **R₄** may be identical or different and are hydrogen, a C₁-C₆-alkylamino group, a C₁-C₆-N,N-dialkylamino group, a C₁-C₆-alkylcyano group, a methoxymethyl group, a tert-butyl group, an isopropyl group, a C₁-C₆-alkyl group, a C₁-C₆-alkyloxy group, a C₁-C₆-hydroxyalkyl group, a C₁-C₆-alkylcarboxylic acid group, a C₁-C₆-alkylcarboxylic acid ester group, a C₁-C₆-alkylcarboxamide group, a C₁-C₆-alkylsulphonic acid group, a C₁-C₆-alkylsulphonic acid ester group, a C₁-C₆-alkylsulphonamide group, a phenyl group or a - (L) -B⁺ group;
**R₅** is a group of the formula (VII) or (VIII); 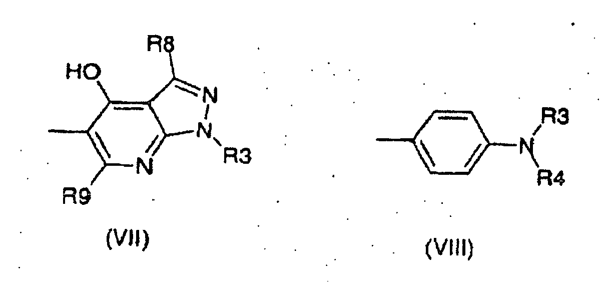
**R₆** is a compound of the formula (IX), (X) or (XI); 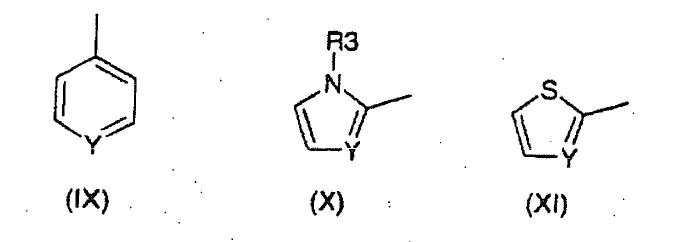
**R₇** is a hydrogen atom, an aliphatic C₁-C₆-alkyl radical, which is linear or branched, unsubstituted or substituted by one or more hydroxyl groups or cationic radicals of type B⁺;
an aromatic radical of the general formula (XII) or (XIII); 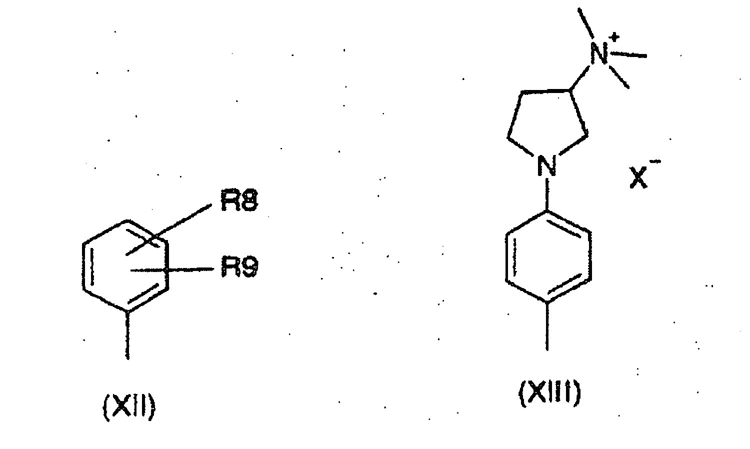 or is a five-membered or six-membered heterocycle or heterocyclic bicycle of the general formula (XIV), (XV), (XVI) or (XVII); 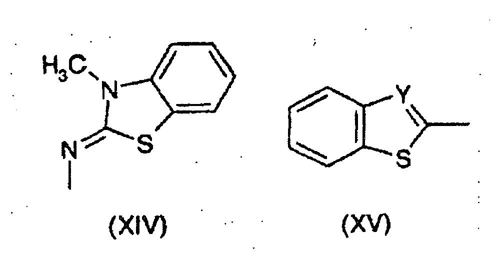 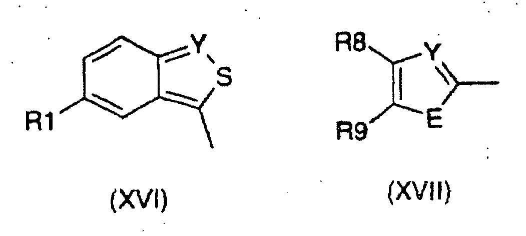
**R₈** and **R₉** may be identical or different and are hydrogen, an amino group, a C₁-C₆-alkylamino group, a C₁-C₆-N, N, -dialkylamino group, a C₁-C₆-N, N- (dihydroxyalkyl)amino group, fluorine, chlorine, bromine, iodine, a cyano groups, a C₁-C₆-alkylcyano group, a methoxymethyl group, a tert-butyl group, an isopropyl group, a C₁-C₆-alkyl group, a C₁-C₆-alkyloxy group, a C₁-C₆-hydroxyalkyl group, a C₁-C₆-hydroxyalkyloxy group, a C₁-C₆-alkylcarboxylic acid group, a C₁-C₆-alkyl-carboxylic acid ester group, a C₁-C₆-alkylcarboxamide group, a C₁-C₆-alkylsulphonic acid group, a C₁-C₆-alkylsulphonic acid ester group, a C₁-C₆-alkylsulphonamide group, a phenyl group, a sulphonic acid group or a -(L)-B⁺ group;
**L** is an C₁-C₆-alkylene group;
**B⁺** is an aromatic heterocyclic quaternary ammonium compound; a nonaromatic heterocyclic quaternary ammonium compound; a quaternary alkylammonium compound or arylammonium compound of the formula NRₐR_{b}R_{c}, where **Rₐ, R_{b},** and **R_{c}**, independently of one another, are a benzyl radical, a phenyl radical or a C₁ to C₆-alkyl radical, where the abovementioned alkyl radicals can be unsubstituted or substituted by one or more hydroxyl groups or amino groups; or
is a quaternary phosphonium group; and **X⁻** is an anion.

2. Agent according to Claim 1, **characterized in that** the naphthalene derivative of the general formula (I) is selected from 3-((E)-{4-[ethyl(2-hydroxyethyl)-amino]phenyl}diazenyl)-8-methyl-7-oxo-7H-benzo[de]-imidazo[1,2-a]quinolin-8-ium methylsulphate, 3-{(E)-[4-hydroxy-1-(2-hydroxyethyl)-3,6-dimethyl-1H-pyrazolo-[3,4-b]pyridin-5-yl]diazenyl}-8-methyl-7-oxo-7H-benzo-[de]imidazo[1,2-a]quinolin-8-ium methylsulphate, 3-methyl-2-(5-nitro-2-oxobenzo[cd]indol-1(2H)-yl-1,3-benzothiazol-3-ium methylsulphate, 3-nitro-7H-benzo-[de]imidazol[1,2-a]quinolin-7-one, 2-(5-(dimethyl-amino)-2-oxobenzo[cd]indol-1-(2H)-yl)-3-methyl-1,3-benzothiazol-3-ium methylsulphate, 3-{2-[4-(5-dimethylamino)-1,3-dioxo-1H-benzo[de]isoquinolin-2(3H)-yl)-(ethyl)anilino]ethyl}-1-methyl-1H-imidazol-3-ium bromide, 5-((E)-{1-[4-dimethylamino)phenyl]-2-oxo-1,2-dihydrobenzo[cd]indol-6-yl}diazenyl)-4-hydroxy-1-(2-hydroxyethyl)-3,6,7-trimethyl-1H-pyrazolo[3,4-b]-pyridin-7-ium methylsulphate, 3-(dimethylamino)-8-methyl-7-oxo-7H-benzo[de]imidazo[1,2-a]quinolin-8-ium methylsulphate, 1-{4-[bis(hydroxyethyl)amino]phenyl}-6-nitrobenzo[cd]indol-2(1H)-one, 3-{2-[ethyl-4-(6-nitro-2-oxobenzo[cd]indol-1(2H)-yl)anilino]ethyl}-1-methyl-1H-imidazol-3-ium bromide, 4-((E)-{[2-(3,4-dimethoxyphenyl)-1,3-dioxo-2,3-dihydro-1H-benzo[de]isoquinolin-6-yl]imino}methyl)-1-(2-hydroxyethyl)pyridinium bromide, 2-(3,4-dimethoxyphenyl)-6-nitro-1H-benzo[de]-isoquinoline-1,3(2H)-dione, 1-(3,4-dimethoxyphenyl)-6-nitrobenzo[cd]indol-2(1H)-one, 1-methyl-3-[2-({2-[((2Z)-3-methyl-1,3-benzothiazol-2(3H)-ylidene)amino]-1,3-dioxo-2,3-dihydro-1H-benzo[de]isoquinolin-6-yl}-amino)ethyl]-1H-imidazol-3-ium bromide, 1-(2-hydroxyethyl)-4-{(E)-[(7-oxo-7H-benzimidazo[2,1-a]benzo[de]-isoquinolin-2-yl)imino]methyl}pyridinium bromide, 1-(2-hydroxyethyl-4-{(E)-(7-thioxo-7H-benzimidazo[2,1-a]benzo[de]isoquinolin-2-yl)imino]methyl}pyridinium bromide, 2-{ethyl-4-[(E)-(14-oxo-14H-benzo[4,5]-isoquinolino[2,1-a]pyrimidin-9-yl)diazenyl]anilino}-N,N,N-trimethylethylethaneaminium bromide, 2-{ethyl-4-[(E)-(7-oxo-7H-benzimidazo[2,1-a]benzo[de]isoquinolin-2-yl)diazenyl]anilino}-N,N,N-trimethylethaneaminium bromide, 2-{ethyl-4-[(E)-(7-thioxo-7H-benzimidazo-[2,1-a]benzo[de]isoquinolin-2-yl)diazenyl]anilino}-N,N,N-trimethylethaneaminium bromide, 2-[4-[(E)-(1,3-dioxo-2,3-dihdyro-1H-benzo[de]isoquinolin-6-yl)diazenyl]-(ethyl)anilino]-N,N,N-trimethylethaneaminium bromide, 1-(2-hydroxyethyl)-4-[(E)-({2-[((2Z)-3-methyl-1,3-benzothiazol-2(3H)-ylidene)amino]-1,3-dioxo-2,3-dihydro-1H-benzo[de]isoquinolin-5-yl}imino)methyl]-pyridinium bromide, 4-{(E)-[(1,3-dioxo-2,3-dihydro-1H-benzo[de]isoquinolin-6-yl)imino]methyl}-1-(2-hydroxyethyl)pyridinium bromide, 2-[4-((E)-{1-[4-(dimethylamino)phenyl]-2-oxo-1,2-dihydrobenzo[cd]indol-5-yl}diazenyl)(ethyl)anilino]-N,N,N-trimethylethaneaminium bromide, 3-{2-[ethyl-4-(2-oxo-5-((E)-{3,4,6-trimethyl-1-[2-(1-methyl-1H-imidazol-3-ium-3-yl)ethyl-1H-pyrazolo[3,4-b)pyridine-5-yl}diazenyl)benzo[cd]-indol-1(2H)-yl)anilino]ethyl}-1-methyl-1H-imidazol-3-ium dibromide, 1-(2-hydroxyethyl)-4-[(E)-({2-[((2Z)-3-methyl-1,3-benzothiazol-2(3H)-ylidene)amino]-1,3-dioxo-2,3-dihydro-1H-benzo[de]isoquinolin-6-yl}imino)methyl]-pyridinium bromide, 2-(6-(dimethylamino)-1,3-dioxo-1H-benzo[de]isoquinolin-2(3H)-yl)-3,4,5-trimethyl-1,3-thiazol-3-ium methylsulphate, 2-(5-(dimethylamino)-2-oxobenzo[cd]indol-1(2H)-yl)-3,4,5-trimethyl-1,3-thaizol-3-ium methylsulphate, 3-{2-[ethyl-4-(6-nitro-1,3-dioxo-1H-benzo[de]isoquinolin-2(3H)-yl)anilino]-ethyl}-1-methyl-1H-imidazol-3-ium bromide, 3-{2-[5-methoxy-2-(6-nitro-1,3-dioxo-1H-benzo[de]isoquinolin-2(3H)-yl)anilino]ethyl}-1-methyl-1H-imidazol-3-ium bromide, 2-[(3-methyl-1,3-benzothiazol-2(3H)-ylidene)-amino]-6-nitro-1H-benzo[de]isoquinoline-1,3(2H)-dione, 2-(1,3-benzothiazol-2-yl)-6-nitro-1H-benzo[de]-isoquinoline-1,3(2H)-dione, 1-(2-hydroxyethyl)-4-{(E)-[(14-oxo-14H-benzo[4,5]isoquino[2,1-a]pyrimidin-9-yl)-imino]methyl}pyridinium bromide and 2-{2-[(2-hydroxyethyl)amino]-4-methoxyphenyl}-5-nitro-1H-benzo-[de]isoquinoline-1,3(2H)-dione.

3. Agent according to Claim 1 or 2, **characterized in that** the naphthalene derivative of the general formula (I) is selected from 3-((E)-{4-ethyl(2-hydroxyethyl)amino]phenyl}diazenyl)-8-methyl-7-oxo-7H-benzo-[de]imidazo[1,2-a]quinolin-8-ium methylsulphate, 3-{(E)-[4-hydroxy-1-(2-hydroxyethyl)-3,6-dimethyl-1H-pyrazolo[3,4-b]pyridin-5-yl]diazenyl}-8-methyl-7-oxo-7H-benzo[de]imidazo[1,2-a]quinolin-8-ium methylsulphate, 3-methyl-2-(5-nitro-2-oxobenzo[cd]indol-1(2H)-yl-1,3-benzothiazol-3-ium methylsulphate, 2-(5-(dimethylamino)-2-oxobenzo[cd]indol-1-(2H)-yl)-3-methyl-1,3-benzothiazol-3-ium methylsulphate, 5-((E)-{1-[4-dimethylamino)phenyl]-2-oxo-1,2-dihydrobenzo-[cd]indol-6-yl}diazenyl)-4-hydroxy-1-(2-hydroxyethyl)-3,6,7-trimethyl-1H-pyrazolo[3,4-b]pyridine-7-ium methylsulphate, 1-{4-[bis(hydroxyethyl)amino]phenyl}-6-nitrobenzo[cd]indol-2(1H)-one, 3-{2-[ethyl-4-(6-nitro-2-oxobenzo[cd]indol-1(2H)-yl)anilino]ethyl}-1-methyl-1H-imidazol-3-ium bromide, 1-(2-hydroxyethyl)-4-{(E)-[(7-oxo-7H-benzimidazo[2,1-a]benzo[de]isoquinolin-2-yl)imino]methyl}pyridinium bromide, 4-{(E)-[(1,3-dioxo-2,3-dihydro-1H-benzo[de]isoquinolin-6-yl)imino]methyl}-1-(2-hydroxy-ethyl)pyridinium bromide, 2-[4-((E)-{1-[4-(dimethylamino)phenyl]-2-oxo-1,2-dihydrobenzo[cd]-indol-5-yl}diazenyl)(ethyl)anilino]-N,N,N-trimethylethaneaminium bromide, 2-[(3-methyl-1,3-benzothiazol-2(3H)-ylidene)amino]-6-nitro-1H-benzo[de]isoquinoline-1,3(2H)-dione, 2-(1,3-benzothiazol-2-yl)-6-nitro-1H-benzo[de]isoquinoline-1,3(2H)-dione, 1-(2-hydroxyethyl)-4-{(E)-[(14-oxo-14H-benzo[4,5]isoquino-[2,1-a]-pyrimidin-9-yl)imino]methyl}pyridinium bromide and 2-{2-[(2-hydroxyethyl)amino]-4-methoxyphenyl}-5-nitro-1H-benzo[de]isoquinoline-1,3(2H)-dione.

4. Agent according to one of Claims 1 to 3,
**characterized in that** it additionally comprises at least one oxidizing agent.

5. Agent according to Claim 4, **characterized in that** the oxidizing agent is selected from hydrogen peroxide or hydrogen peroxide adducts, persulphates and perborates.

6. Agent according to one of Claims 1 to 3,
**characterized in that** it comprises at least one natural or synthetic polymer or modified polymer of natural origin customary for cosmetic agents.

7. Agent according to Claim 6, **characterized in that** it comprises the polymer in an amount of from 1 to 5% by weight.

8. Agent according to one of Claims 1 to 7,
**characterized in that** it comprises the naphthalene derivative of the general formula (I) in an amount of from 0.01 to 10% by weight.

9. Agent according to one of Claims 1 to 8,
**characterized in that** it is a hair dye.

10. Naphthalene derivative, **characterized in that** it is selected from 3-((E)-{4-[ethyl(2-hydroxyethyl)-amino]phenyl}diazenyl)-8-methyl-7-oxo-7H-benzo[de]-imidazo[1,2-a]quinolin-8-ium methylsulphate, 3-{(E)-[4-hydroxy-1-(2-hydroxyethyl)-3,6-dimethyl-1H-pyrazolo-[3,4-b]pyridin-5-yl]diazenyl}-8-methyl-7-oxo-7H-benzo-[de]imidazo[1,2-a]quinolin-8-ium methylsulphate, 3-methyl-2-(5-nitro-2-oxobenzo[cd]indol-1(2H)-yl-1,3-benzothiazol-3-ium methylsulphate, 3-nitro-7H-benzo[de]imidazol[1,2-a]quinolin-7-one, 2-(5-(dimethylamino)-2-oxobenzo[cd]indol-1-(2H)-yl)-3-methyl-1,3-benzothiazol-3-ium methylsulphate, 3-{2-[4-(5-dimethylamino)-1,3-dioxo-1H-benzo[de]isoquinolin-2(3H)-yl)-(ethyl)anilino]ethyl}-1-methyl-1H-imidazol-3-ium bromide, 5-((E)-{1-[4-dimethylamino)phenyl]-2-oxo-1,2-dihydrobenzo[cd]indol-6-yl}diazenyl)-4-hydroxy-1-(2-hydroxyethyl)-3,6,7-trimethyl-1H-pyrazolo[3,4-b]-pyridin-7-ium methylsulphate, 3-(dimethylamino)-8-methyl-7-oxo-7H-benzo[de]imidazo[1,2-a]quinolin-8-ium methylsulphate, 1-{4-[bis(hydroxyethyl)amino]phenyl}-6-nitrobenzo[cd]indol-2(1H)-one, 3-{2-[ethyl-4-(6-nitro-2-oxobenzo[cd]indol-1(2H)-yl)anilino]ethyl}-1-methyl-1H-imidazol-3-ium bromide, 4-((E)-{[2-(3,4-dimethoxyphenyl)-1,3-dioxo-2,3-dihydro-1H-benzo[de]isoquinolin-6-yl]imino}methyl)-1-(2-hydroxyethyl)pyridinium bromide, 2-(3,4-dimethoxyphenyl)-6-nitro-1H-benzo[de]-isoquinoline-1,3(2H)-dione, 1-(3,4-dimethoxyphenyl)-6-nitrobenzo[cd]indol-2(1H)-one, 1-methyl-3-[2-({2-[((2Z)-3-methyl-1,3-benzothiazol-2(3H)-ylidene)amino]-1,3-dioxo-2,3-dihydro-1H-benzo[de]isoquinolin-6-yl}-amino)ethyl]-1H-imidazol-3-ium bromide, 1-(2-hydroxyethyl)-4-{(E)-[(7-oxo-7H-benzimidazo[2,1-a]benzo[de]-isoquinolin-2-yl)imino]methyl}pyridinium bromide, 1-(2-hydroxyethyl-4-{(E)-(7-thioxo-7H-benzimidazo[2,1-a]-benzo[de]isoquinolin-2-yl)imino]methyl}pyridinium bromide, 2-{ethyl-4-[(E)-(14-oxo-14H-benzo[4,5]-isoquinolino[2,1-a]pyrimidin-9-yl)diazenyl]anilino}-N,N,N-trimethylethylethaneaminium bromide, 2-{ethyl-4-[(E)-(7-oxo-7H-benzimidazo[2,1-a]benzo[de]isoquinolin-2-yl)diazenyl]anilino}-N,N,N-trimethylethaneaminium bromide, 2-{ethyl-4-[(E)-(7-thioxo-7H-benzimidazo-[2,1-a]benzo[de]isoquinolin-2-yl)diazenyl]anilino}-N,N,N-trimethylethaneaminium bromide, 2-[4-[(E)-(1,3-dioxo-2,3-dihdyro-1H-benzo[de]isoquinolin-6-yl)diazenyl]-(ethyl)anilino]-N,N,N-trimethylethaneaminium bromide, 1-(2-hydroxyethyl)-4-[(E)-({2-[((2Z)-3-methyl-1,3-benzothiazol-2(3H)-ylidene)amino]-1,3-dioxo-2,3-dihydro-1H-benzo[de]isoquinolin-5-yl}imino)methyl]-pyridinium bromide, 4-{(E)-[(1,3-dioxo-2,3-dihydro-1H-benzo[de]isoquinolin-6-yl)imino]methyl}-1-(2-hydroxyethyl)pyridinium bromide, 2-[4-((E)-{1-[4-(dimethylamino)phenyl]-2-oxo-1,2-dihydrobenzo[cd]indol-5-yl}-diazenyl)(ethyl)anilino]-N,N,N-trimethylethaneaminium bromide, 3-{2-[ethyl-4-(2-oxo-5-((E)-{3,4,6-trimethyl-1-[2-(1-methyl-1H-imidazol-3-ium-3-yl)ethyl-1H-pyrazolo[3,4-b)pyridine-5-yl}diazenyl)benzo[cd]indol-1(2H)-yl)anilino]ethyl}-1-methyl-1H-imidazol-3-ium dibromide, 1-(2-hydroxyethyl)-4-((E)-({2-[((2Z)-3-methyl-1,3-benzothiazol-2(3H)-ylidene)amino]-1,3-dioxo-2,3-dihydro-1H-benzo[de]isoquinolin-6-yl}imino)methyl]-pyridinium bromide, 2-(6-(dimethylamino)-1,3-dioxo-1H-benzo[de]isoquinolin-2(3H)-yl)-3,4,5-trimethyl-1,3-thiazol-3-ium methylsulphate, 2-(5-(dimethylamino)-2-oxobenzo[cd]indol-1(2H)-yl)-3,4,5-trimethyl-1,3-thaizol-3-ium methylsulphate, 3-{2-[ethyl-4-(6-nitro-1,3-dioxo-1H-benzo[de]isoquinolin-2(3H)-yl)anilino]-ethyl}-1-methyl-1H-imidazol-3-ium bromide, 3-{2-[5-methoxy-2-(6-nitro-1,3-dioxo-1H-benzo[de]isoquinolin-2(3H)-yl)anilino]ethyl}-1-methyl-1H-imidazol-3-ium bromide, 2-[(3-methyl-1,3-benzothiazol-2(3H)-ylidene)-amino]-6-nitro-1H-benzo[de]isoquinoline-1,3(2H)-dione, 2-(1,3-benzothiazol-2-yl)-6-nitro-1H-benzo[de]-isoquinoline-1,3(2H)-dione, 1-(2-hydroxyethyl)-4-{(E)-[(14-oxo-14H-benzo[4,5]isoquino[2,1-a]pyrimidin-9-yl)-imino]methyl}pyridinium bromide and 2-{2-[(2-hydroxyethyl)amino]-4-methoxyphenyl}-5-nitro-1H-benzo-[de]isoquinoline-1,3(2H)-dione.

11. Naphthalene derivative according to Claim 10, **characterized in that** it is selected from 3-((E)-{4-ethyl(2-hydroxyethyl)amino]phenyl}diazenyl)-8-methyl-7-oxo-7H-benzo[de]imidazo[1,2-a]quinolin-8-ium methylsulphate, 3-{(E)-[4-hydroxy-1-(2-hydroxyethyl)-3,6-dimethyl-1H-pyrazolo[3,4-b]pyridin-5-yl]diazenyl}-8-methyl-7-oxo-7H-benzo[de]imidazo[1,2-a]quinolin-8-ium methylsulphate, 3-methyl-2-(5-nitro-2-oxobenzo[cd]-indol-1(2H)-yl-1,3-benzothiazol-3-ium methylsulphate, 2-(5-(dimethylamino)-2-oxobenzo[cd]indol-1-(2H)-yl)-3-methyl-1,3-benzothiazol-3-ium methylsulphate, 5-((E)-{1-[4-dimethylamino)phenyl]-2-oxo-1,2-dihydrobenzo[cd]-indol-6-yl}diazenyl)-4-hydroxy-1-(2-hydroxyethyl)-3,6,7-trimethyl-1H-pyrazolo[3,4-b]pyridine-7-ium methylsulphate, 1-{4-[bis(hydroxyethyl)amino]phenyl}-6-nitrobenzo[cd]indol-2(1H)-one, 3-{2-[ethyl-4-(6-nitro-2-oxobenzo[cd]indol-1(2H)-yl)anilino]ethyl}-1-methyl-1H-imidazol-3-ium bromide, 1-(2-hydroxyethyl)-4-{(E)-[(7-oxo-7H-benzimidazo[2,1-a]benzo[de]isoquinolin-2-yl)imino]methyl}pyridinium bromide, 4-{(E)-[(1,3-dioxo-2,3-dihydro-1H-benzo[de]isoquinolin-6-yl)imino]methyl}-1-(2-hydroxy-ethyl)pyridinium bromide, 2-[4-((E)-{1-[4-(dimethylamino)phenyl]-2-oxo-1,2-dihydrobenzo[cd]-indol-5-yl}diazenyl)(ethyl)anilino]-N,N,N-trimethylethaneaminium bromide, 2-[(3-methyl-1,3-benzothiazol-2(3H)-ylidene)amino]-6-nitro-1H-benzo[de]isoquinoline-1,3(2H)-dione, 2-(1,3-benzothiazol-2-yl)-6-nitro-1H-benzo[de]isoquinoline-1,3(2H)-dione, 1-(2-hydroxyethyl)-4-{(E)-[(14-oxo-14H-benzo[4,5]isoquino-[2,l-a]-pyrimidin-9-yl)imino]methyl}pyridinium bromide and 2-{2-[(2-hydroxyethyl)amino]-4-methoxyphenyl}-5-nitro-1H-benzo[de]isoquinoline-1,3(2H)-dione.

## Revendications

1. Composition pour la teinture de fibres de kératine, **caractérisée en ce qu'**elle contient au moins un dérivé de naphtalène de formule générale (I) 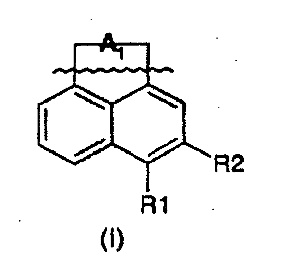 dans laquelle
**A₁** représente une structure partielle de formules (II), (III), (IV), (V) et (VI) 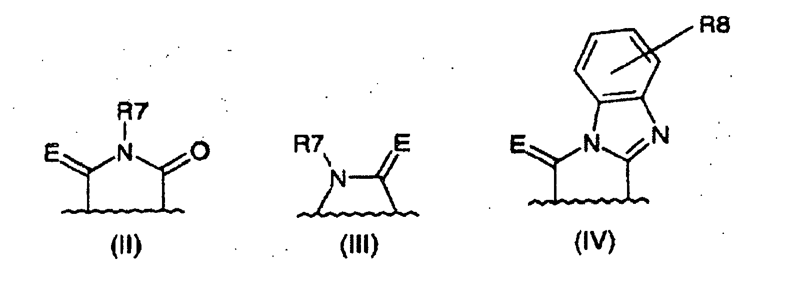 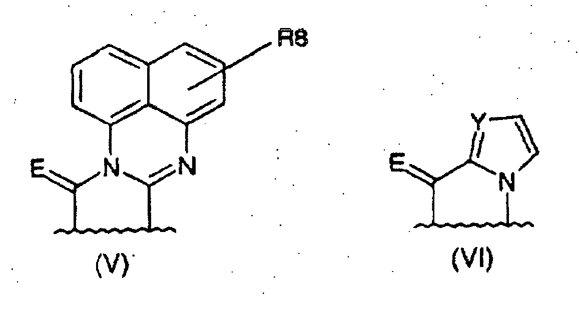 dans lesquelles
**E** représente un atome d'oxygène ou un atome de soufre ;
**Y** représente un atome d'azote ou un atome d'azote quaternaire, qui est substitué par des groupes alkyle en C₁-C₆ linéaires ou ramifiés, des groupes hydroxyalkyle en C₂-C₄ linéaires ou ramifiés ou des groupes polyhydroxyalkyle en C₄-C₆ linéaires ou ramifiés ;
**R₁** et **R₂** représentent, indépendamment l'un de l'autre, un atome d'hydrogène, un groupe -N-R₃R₄, un groupe nitro, un groupe -N=N-R₅ ou un groupe -N=C-R₃R₆ ;
**R₃** et **R₄** peuvent être identiques ou différents et représentent un atome d'hydrogène, un groupe alkyl(C₁-C₆) amino, un groupe N,N-dialkyl (C₁-C₆)-amino, un groupe alkyl(C₁-C₆)cyano, un groupe méthoxyméthyle, un groupe tert-butyle, un groupe isopropyle, un groupe alkyle en C₁-C₆, un groupe alkyloxy en C₁-C₆, un groupe hydroxyalkyle en C₁-C₆, un groupe acide alkyl (C₁-C₆) carboxylique, un groupe ester d'acide alkyl (C₁-C₆)carboxylique, un groupe alkyl(C₁-C₆)carboxamido, un groupe acide alkyl(C₁-C₆)sulfonique, un groupe ester d'acide alkyl(C₁-C₆)sulfonique, un groupe alkyl(C₁-C₆) - sulfonamido, un groupe phényle ou un groupe -(L)-B⁺;
**R₅** représente un groupe de formule (VII) ou (VIII) ; 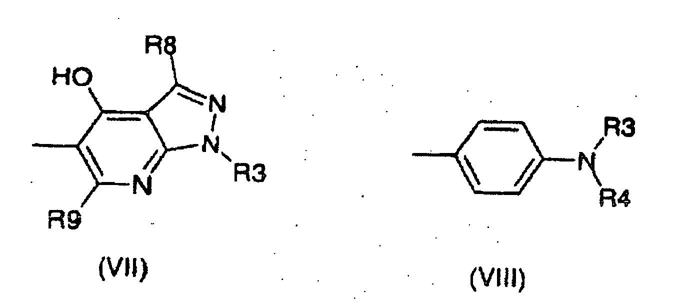
**R₆** représente un composé de formule (IX), (X) ou (XI) ; 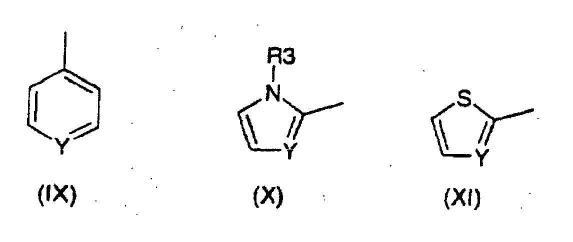
**R₇** représente un atome d'hydrogène, un radical aliphatique alkyle en C₁-C₆ qui est linéaire ou ramifié, non substitué ou substitué par un ou plusieurs groupes hydroxy ou radicaux cationiques du type B⁺ ;
un radical aromatique de formule générale (XII) ou (XIII) ; 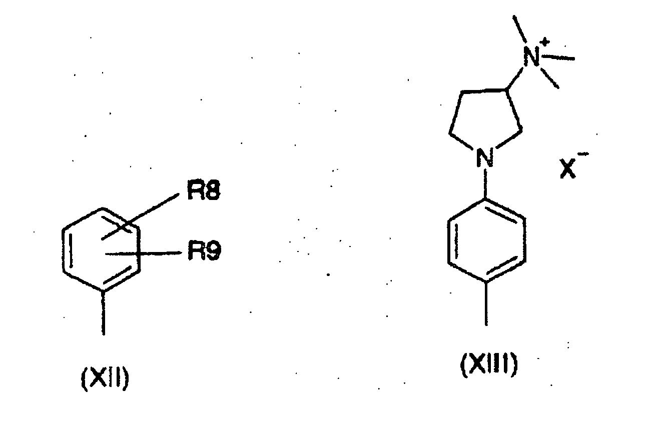 ou un hétérocycle monocyclique ou bicyclique à cinq chaînons ou à six chaînons de formules générales (XIV), (XV), (XVI) ou (XVII) ; 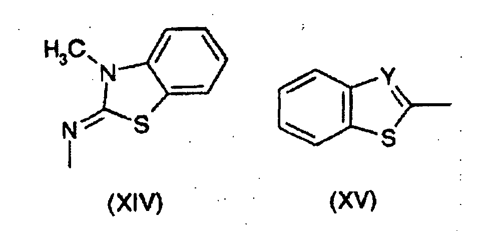 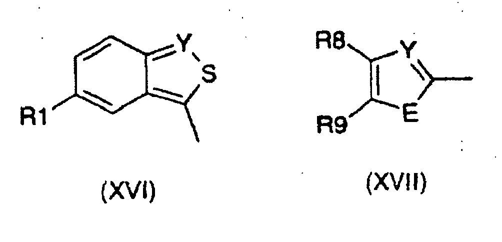
**R₈** et **R₉** peuvent être identiques ou différents et représentent un atome d'hydrogène, un groupe amino, un groupe alkyl(C₁-C₆)amino, un groupe N,N-dialkyl(C₁-C₆)amino, un groupe N,N-[dihydroxyalkyl-(C₁-C₆)]amino, un atome de fluor, chlore, brome, iode, un groupe cyano, un groupe alkyl(C₁-C₆)cyano, un groupe méthoxyméthyle, un groupe tert-butyle, un groupe isopropyle, un groupe alkyle en C₁-C₆, un groupe alkyloxy en C₁-C₆, un groupe hydroxyalkyle en C₁-C₆, un groupe hydroxyalkyloxy en C₁-C₆, un groupe acide alkyl(C₁-C₆) carboxylique, un groupe ester d'acide alkyl(C₁-C₆)carboxylique, un groupe alkyl (C₁-C₆) carboxamido, un groupe acide alkyl(C₁-C₆)sulfonique, un groupe ester d'acide alkyl(C₁-C₆)sulfonique, un groupe alkyl (C₁-C₆) - sulfonamido, un groupe phényle, un groupe acide sulfonique ou un groupe -(L)-B⁺ ;
**L** représente un groupe alcényle en C₁-C₆ ;
**B⁺** représente un composé , ammonium quaternaire hétérocyclique aromatique ; un composé ammonium quaternaire hétérocyclique non aromatique ; un composé alkylammonium quaternaire ou un composé arylammonium de formule NRₐR_{b}R_{c}, **Rₐ, R_{b}** et **R_{c}** représentant chacun indépendamment un radical benzyle, un radical phényle ou un radical alkyle en C₁-C₆, les radicaux alkyle précités pouvant être non substitués ou substitués par un ou plusieurs groupes hydroxy ou amino ; ou
un groupe phosphonium quaternaire ; et **X⁻** est un anion.

2. Composition selon la revendication 1, **caractérisée en ce que** le dérivé de naphtalène de formule générale (I) est choisi parmi le méthylsulfate de 3-((E)-{4-[éthyl(2-hydroxyéthyl)amino]phényl}-diazényl)-8-méthyl-7-oxo-7H-benzo[de]-imidazo-[1,2-a]quinoléin-8-ium, le méthylsulfate de 3-{(E)-[4-hydroxy-1-(2-hydroxyéthyl)-3,6-diméthyl-1H-pyrazolo[3,4-b]pyridin-5-yl]diazényl}-8-méthyl-7-oxo-7H-benzo[de]imidazo[1,2-a]quinoléin-8-ium, le méthylsulfate de 3-méthyl-2-(5-nitro-2-oxobenzo[cd]indol-1(2H)-yl)-1,3-benzothiazol-3-ium, la 3-nitro-7H-benzo[de]imidazo[1,2-a]-quinoléin-7-one, le méthylsulfate de 2-(5-(diméthylamino)-2-oxobenzo[cd]indol-1(2H)-yl)-3-méthyl-1,3-benzothiazol-3-ium, le bromure de 3-{2-[4-(5-(diméthylamino)-1,3-dioxo-1H-benzo-[de]isoquinoléin-2(3H)-yl)(éthyl)anilino]éthyl}-1-méthyl-1H-imidazol-3-ium, le méthylsulfate de 5-((E)-{1-[4-(diméthylamino)phényl]-2-oxo-1,2-dihydrobenzo[cd]indol-6-yl}diazényl)-4-hydroxy-1-(2-hydroxyéthyl)-3,6,7-triméthyl-1H-pyrazolo[3,4-b]pyridin-7-ium, le méthylsulfate de 3-(diméthylamino)-8-méthyl-7-oxo-7H-benzo[de]-imidazo[1,2-a]quinolin-8-ium-, la 1-{4-[bis-(hydroxyéthyl)amino]phényl}-6-nitrobenzo[cd]indol-2(1H)-one, le bromure de 3-{2-[éthyl-4-(6-nitro-2-oxobenzo[cd]indol-1(2H)-yl)anilino]éthyl}-1-méthyl-1H-imidazol-3-ium, le bromure de 4-((E)-{[2-(3,4-diméthoxyphényl)-1,3-dioxo-2,3dihydro-1H-benzo[de]isoquinolin-6-yl]imino}méthyl)-1-(2-hydroxyéthyl)pyridinium, la 2-(3,4-diméthoxyphényl)-6-nitro-1H-benzo[de]isoquinoléine-1,3(2H)-dione, la 1-(3,4-diméthoxyphényl)-6-nitrobenzo-[cd]indol-2(1H)-one, le bromure de 1-méthyl-3-[2-({2-[((2Z)-3-méthyl-1,3-benzothiazol-2(3H)-ylidène)amino]-1,3-dioxo-2,3-dihydro-1H-benzo[de]isoquinoléin-6-yl}amino)éthyl]-1H-imidazol-3-ium, le bromure de 1-(2-hydroxyéthyl)-4-{(E)-[(7-oxo-7H-benzimidazo[2,1-a]benzo[de]-isoquinoléin-2-yl)imino]méthyl}pyridinium, le bromure de 1-(2-hydroxyéthyl-4-{(E)-[7-thioxo-7H-benzimidazo[2,1-a]benzo[de]isoquinoléin-2-yl)-imino]méthyl}-pyridinium, le bromure de 2-{éthyl-4-[(E)-(14-oxo-14H-benzo[4,5]isoquinoléino[2,1-a)pyrimidin-9-yl)diazényl]anilino}-N,N,N-triméthyléthyléthanaminium, le bromure de 2-{éthyl-4-[(E)-(7-oxo-7H-benzimidazo[2,1-a]benzo[de]-isoquinoléin-2-yl)diazényl]anilino)-N,N,N-triméthyléthanaminium, le bromure de 2-{éthyl-4-[(E)-(7-thioxo-7H-benzimidazo-[2,1-a]benzo[de]-isoquinoléin-2-yl)diazényl]anilino}-N,N,N-triméthyléthanaminium, le bromure de 2-[4-[(E)-(1,3-dioxo-2,3-dihydro-1H-benzo[de]isoquinoléin-6-yl)diazényl](éthyl)anilino]-N,N,N-triméthyléthanaminium, le bromure de 1-(2-hydroxyéthyl)-4-[(E)-({2-[((2Z)-3-méthyl-1,3-benzothiazol-2(3H)-ylidène)amino]-1,3-dioxo-2,3-dihydro-1H-benzo[de]-isoquinoléin-5-yl}imino)méthyl]pyridinium, le bromure de 4-{(E)-[(1,3-dioxo-2,3-dihydro-1H-benzo[de]isoquinoléin-6-yl)imino]méthyl}-1-(2-hydroxyéthyl)pyridinium, le bromure de 2-[4-((E)-{1-[4-(diméthylamino)phényl]-2-oxo-1,2-dihydrobenzo[cd]indol-5-yl}diazényl)(éthyl)anilino]-N,N,N-triméthyléthanaminium, le dibromure de 3-{2-[éthyl-4-(2-oxo-5-((E)-{3,4,6-triméthyl-1-[2-(1-méthyl-1H-imidazol-3-ium-3-yl)éthyl-1H-pyrazolo-[3,4-b]pyridin-5-yl}diazényl)benzo[cd]indol-1(2H)-yl)anilino]éthyl}-1-méthyl-1H-imidazol-3-ium, le bromure de 1-(2-hydroxyéthyl)-4-[(E)-({2-[((2Z)-3-méthyl-1,3-benzothiazol-2(3H)-ylidène)amino]-1,3-dioxo-2,3-dihydro-1H-benzo[de]isoquinoléin-6-yl}imino)méthyl]pyridinium, le méthylsulfate de 2-(6-(diméthylamino)-1,3-dioxo-1H-benzo[de]-isoquinoléin-2(3H)-yl)-3,4,5-triméthyl-1,3-thiazol-3-ium, le méthylsulfate de 2-(5-(diméthylamino)-2-oxobenzo[cd]indol-1(2H)-yl)-3,4,5-triméthyl-1,3-thiazol-3-ium, le bromure de 3-{2-[éthyl-4-(6-nitro-1,3-dioxo-1H-benzo[de]isoquinoléine-2(3H)-yl)anilino]éthyl}-1-méthyl-1H-imidazol-3-ium, le bromure de 3-{2-[5-méthoxy-2-(6-nitro-1,3-dioxo-1H-benzo[de]-isoquinoléin-2(3H)-yl)anilino]éthyl}-1-méthyl-1H-imidazol-3-ium, la 2-[(3-méthyl-1,3-benzothiazol-2(3H)-ylidène)amino]-6-nitro-1H-benzo[de]-isoquinoléine-1,3(2H)-dione, la 2-(1,3-benzothiazol-2-yl)-6-nitro-1H-benzo[de]-isoquinoléin-1,3(2H)-dione, le bromure de 1-(2-hydroxyéthyl)-4-{(E)-[(14-oxo-14H-benzo[4,5]-isoquino[2,1-a]pyrimidin-9-yl)imino]méthyl}-pyridinium et la 2-{2-[(2-hydroxyéthyl)amino]-4-méthoxyphényl}-5-nitro-1H-benzo[de]isoquinoléine-1,3(2H)-dione.

3. Composition selon la revendication 1 ou 2, **caractérisée en ce que** le dérivé de naphtalène de formule générale (I) est choisi parmi le méthylsulfate de 3-((E)-{4-[éthyl(2-hydroxyéthyl)-amino]phényl}diazényl)-8-méthyl-7-oxo-7H-benzo-[de)-imidazo-[1,2-a]quinoléine-8-ium, le méthylsulfate de 3-{(E)-[4-hydroxy-1-(2-hydroxyéthyl)-3,6-diméthyl-1H-pyrazolo[3,4-b]pyridin-5-yl]diazényl}-8-méthyl-7-oxo-7H-benzo[de]imidazo-[1,2-a]quinoléin-8-ium, le méthylsulfate de 3-méthyl-2- (5-nitro-2-oxobenzo [cd] indol-1 (2H)-yl) - 1,3-benzothiazol-3-ium, le méthylsulfate de 2-(5-(diméthylamino)-2-oxobenzo[cd]indol-1(2H)-yl)-3-méthyl-1,3-benzothiazol-3-ium, le méthylsulfate de 5-((E)-{1-[4-(diméthylamino)phényl]-2-oxo-1,2-dihydrobenzo[cd]indol-6-yl}diazényl)-4-hydroxy-l-(2-hydroxyéthyl)-3,6,7-triméthyl-1H-pyrazolo[3,4-b]pyridin-7-ium, la 1-{4-[bis(hydroxyéthyl)-amino]phényl}-6-nitrobenzo[cd]indol-2(1H)-one, le bromure de 3-{2-[éthyl-4-(6-nitro-2-oxobenzo[cd]-indol-l(2H)-yl)anilino]êthyl}-l-méthyl-1H-imidazol-3-ium, le bromure de 1-(2-hydroxyéthyl)-4-{(E)-[(7-oxo-7H-benzimidazo[2,1-a]benzo[de]-isoquinoléin-2-yl)imino]méthyl}pyridinium, le bromure de 4-{(E)-[(1,3-dioxo-2,3-dihydro-1H-benzo[de]isoquinoléin-6-yl)imino]méthyl}-1-(2-hydroxyéthyl)pyridinium, le bromure de 2-[4-((E)-{1-[4-(diméthylamino)phényl]-2-oxo-1,2-dihydrobenzo[cd]indol-5-yl}diazényl)(éthyl)aniline]-N,N,N-triméthyléthanaminium, la 2-[(3-méthyl-1,3-benzothiazol-2(3H)-ylidène)amino]-6-nitro-1H-benzo[de]isoquinoléine-1,3(2H)-dione, la 2-(1,3-benzothiazol-2-yl)-6-nitro-1H-benzo[de]-isoquinoléine-1,3(2H)-dione, le bromure de 1-(2-hydroxyéthyl)-4-{(E)-[(14-oxo-14H-benzo[4,5] - isoquino[2,1-a]pyrimidin-9-yl)imino]méthyl}-pyridinium et la 2-{2-[(2-hydroxyéthyl)amino]-4-méthoxyphényl}-5-nitro-1H-benzo[de]isoquinoléine-1,3(2H)-dione.

4. Composition selon l'une quelconque des revendications 1 à 3, **caractérisée en ce qu'**elle contient en outre au moins un oxydant.

5. Composition selon la revendication 4, **caractérisée en ce que** l'oxydant est choisi parmi le peroxyde d'hydrogène ou des adduits de peroxyde d'hydrogène, des persulfates et des perborates.

6. Composition selon l'une quelconque des revendications 1 à 3, **caractérisée en ce qu'**elle contient au moins un polymère naturel ou synthétique ou un polymère modifié d'origine naturelle, usuel pour des produits cosmétiques.

7. Composition selon la revendication 6, **caractérisée en ce qu'**elle contient le polymère en une quantité de 1 à 5 % en poids.

8. Composition selon l'une quelconque des revendications 1 à 7, **caractérisée en ce qu'**elle contient le dérivé de naphtalène de formule générale (I) en une quantité de 0,01 à 10 % en poids.

9. Composition selon l'une quelconque des revendications 1 à 8, **caractérisée en ce qu'**elle est une composition de teinture pour cheveux.

10. Dérivé de naphtalène, **caractérisé en ce qu'**il est choisi parmi le méthylsulfate de 3-((E)-{4-[éthyl(2-hydroxyéthyl)amino]phényl}diazényl)-8-méthyl-7-oxo-7H-benzo[de]-imidazo-[1,2-a] quinoléin-8-ium, le méthylsulfate de 3-{(E)-[4-hydroxy-1-(2-hydroxyéthyl)-3,6-diméthyl-1H-pyrazolo[3,4-b]pyridin-5-yl]diazényl}-8-méthyl-7-oxo-7H-benzo[de]imidazo[1,2-a]quinoléin-8-ium, le méthylsulfate de 3-méthyl-2-(5-nitro-2-oxobenzo-[cd]indol-1(2H)-yl)-1,3-benzothiazol-3-ium, la 3-nitro-7H-benzo[de]imidazo[1,2-a]quinoléin-7-one, le méthylsulfate de 2-(5-(diméthylamino)-2-oxobenzo[cd]indol-1(2H)-yl)-3-méthyl-1,3-benzothiazol-3-ium, le bromure de 3-{2-[4-(5-(diméthylamino)-1,3-dioxo-1H-benzo-[de]isoquinoléin-2(3H)-yl) (éthyl)anilino]éthyl}-1-méthyl-1H-imidazol-3-ium, le méthylsulfate de 5-((E)-{1-[4-(diméthylamino)phényl]-2-oxo-1,2-dihydrobenzo[cd]indol-6-yl}diazényl)-4-hydroxy-1-(2-hydroxyéthyl)-3,6,7-triméthyl-1H-pyrazolo[3,4-b]pyridin-7-ium, le méthylsulfate de 3-(diméthylamino)-8-méthyl-7-oxo-7H-benzo[de]-imidazo[1,2-a]quinoléin-8-ium, la 1-{4-(bis-(hydroxyéthyl)amino]phényl}-6-nitrobenzo[cd]indol-2(1H)-one, le bromure de 3-{2-[éthyl-4-(6-nitro-2-oxobenzo[cd]indol-1(2H)-yl)anilino]éthyl}-1-méthyl-1H-imidazol-3-ium, le bromure de 4-((E)-{[2-(3,4-diméthoxyphényl)-1,3-dioxo-2,3-dihydro-1H-benzo[de]isoquinoléin-6-yl]imino}méthyl)-1-(2-hydroxyéthyl)pyridinium, la 2-(3,4-diméthoxyphényl)-6-nitro-1H-benzo[de]isoquinoléin-1,3(2H)-dione, la 1-(3,4-diméthoxyphényl)-6-nitrobenzo-[cd]indol-2(1H)-one, le bromure de 1-méthyl-3-[2-({2-[((2Z)-3-méthyl-1,3-benzothiazol-2(3H)-ylidène)amino]-1,3-dioxo-2,3-dihydro-1H-benzo[de]isoquinolin-6-yl}amino)éthyl]-1H-imidazol-3-ium, le bromure de 1-(2-hydroxyéthyl)-4-{(E)-[(7-oxo-7H-benzimidazo[2,1-a]benzo[de]-isoquinoléin-2-yl)imino]méthyl}pyridinium, le bromure de 1-(2-hydroxyéthyl-4-{(E)-[7-thioxo-7H-benzimidazo[2,1-a]benzo[de]isoquinoléin-2-yl)-imino]méthyl}-pyridinium, le bromure de 2-{éthyl-4-[(E)-(14-oxo-14H-benzo[4,5]isoquinoléino[2,1-a]pyrimidin-9-yl)diazényl]anilino}-N,N,N-triméthyléthyléthanaminium, le bromure de 2-{éthyl-4-[(E)-(7-oxo-7H-benzimidazo[2,1-a]benzo[de]-isoquinoléin-2-yl)diazényl]anilino]-N,N,N-triméthyléthanaminium, le bromure de 2-{éthyl-4-[(E)-(7-thioxo-7H-benzimidazo-[2,l-a]benzo[de]-isoquinoléin-2-yl)diazényl]anilino}-N,N,N-triméthyléthanaminium, le bromure de 2-[4-[(E)-(1,3-dioxo-2,3-dihydro-1H-benzo[de]isoquinoléin-6-yl)diazényl](éthyl)anilino]-N,N,N-triméthyléthanaminium, le bromure de 1-(2-hydroxyéthyl)-4-[(E)-({2-[((2Z)-3-méthyl-1,3-benzothiazol-2(3H)-ylidène)amino]-1,3-dioxo-2,3-dihydro-1H-benzo[de]-isoquinoléin-5-yl}imino)méthyl]pyridinium, le bromure de 4-{(E)-[(1,3-dioxo-2,3-dihydro-1H-benzo[de]isoquinoléin-6-yl)imino]méthyl}-1-(2-hydroxyéthyl)pyridinium, le bromure de 2-[4-((E)-{1-[4-(diméthylamino)phényl]-2-oxo-1,2-dihydrobenzo [cd] indol-5-yl}diazényl) (éthyl) anilino] - N,N,N-triméthyléthanaminium, le dibromure de 3-{2-[éthyl-4-(2-oxo-5-((E)-{3,4,6-triméthyl-1-[2-(1-méthyl-1H-imidazol-3-ium-3-yl)éthyl-1H-pyrazolo-[3,4-b]pyridin-5-yl}diazényl)benzo[cd]indol-1(2H)-yl)anilino]éthyl}-1-méthyl-1H-imidazol-3-ium, le bromure de1-(2-hydroxyéthyl)-4-[(E)-({2-[((2Z)-3-méthyl-1,3-benzothiazol-2(3H)-ylidène)amino]-1,3-dioxo-2,3-dihydro-1H-benzo[de]isoquinoléin-6-yl}imino)méthyl]pyridinium, le méthylsulfate de 2-(6-(diméthylamino)-1,3-dioxo-1H-benzo[de]-isoquinoléin-2(3H)-yl)-3,4,5-triméthyl-1,3-thiazol-3-ium, le méthylsulfate de 2-(5-(diméthylamino)-2-oxobenzo[cd]indol-1(2H)-yl)-3,4,5-triméthyl-1,3-thiazol-3-ium, le bromure de 3-{2-[éthyl-4-(6-nitro-1,3-dioxo-1H-benzo[de]isoquinoléin-2(3H)-yl)anilino]éthyl}-1-méthyl-1H-imidazol-3-ium, le bromure de 3-{2-[5-méthoxy-2-(6-nitro-1,3-dioxo-1H-benzo[de]-isoquinolién-2(3H)-yl)anilino]éthyl}-1-méthyl-1H-imidazol-3-ium, la 2-[(3-méthyl-1,3-benzothiazol-2(3H)-ylidène)amino]-6-nitro-1H-benzo[de]-isoquinoléine-1,3(2H)-dione, la 2-(1,3-benzothiazol-2-yl)-6-nitro-1H-benzo[de]-isoquinoléin-1,3(2H)-dione, le bromure de 1-(2-hydroxyéthyl)-4-{(E)-[(14-oxo-14H-benzo[4,5]-isoquino[2,1-a]pyrimidin-9-yl)imino]méthyl}-pyridinium et la 2-{2-[(2-hydroxyéthyl)amino]-4-méthoxyphényl}-5-nitro-1H-benzo[de]isoquinoléine-1,3(2H)-dione.

11. Dérivé de naphtalène selon la revendication 10, **caractérisé en ce qu'**il est choisi parmi le méthylsulfate de 3-((E)-{4-[éthyl(2-hydroxyéthyl)-amino]phényl}diazényl)-8-méthyl-7-oxo-7H-benzo-[de]-imidazo-[1,2-a]quinoléin-8-ium, le méthylsulfate de 3-{(E)-[4-hydroxy-1-(2-hydroxyéthyl)-3,6-diméthyl-1H-pyrazolo[3,4-b]pyridin-5-yl]-diazényl}-8-méthyl-7-oxo-7H-benzo[de]imidazo[1,2-a]quinoléin-8-ium, le méthylsulfate de 3-méthyl-2-(5-nitro-2-oxobenzo[cd]indol-1(2H)-yl)-1,3-benzothiazol-3-ium, le méthylsulfate de 2-(5-(diméthylamino)-2-oxobenzo[cd]indol-1(2H)-yl)-3-méthyl-1,3-benzothiazol-3-ium, le méthylsulfate de 5-((E)-{1-[4-(diméthylamino)phényl]-2-oxo-1,2-dihydrobenzo[cd]indol-6-yl}diazényl)-4-hydroxy-1-(2-hydroxyéthyl)-3,6,7-triméthyl-1H-pyrazolo[3,4-b]pyridin-7-ium, la 1-{4-[bis(hydroxyéthyl)-amino]phényl}-6-nitrobenzo[cd]indol-2(1H)-one, le bromure de 3-{2-[éthyl-4-(6-nitro-2-oxobenzo[cd]-indol-1(2H)-yl)anilino]éthyl}-1-méthyl-1H-imidazol-3-ium, le bromure de 1-(2-hydroxyéthyl)-4-{(E)-[(7-oxo-7H-benzimidazo[2,1-a]benzo[de]-isoquinoléin-2-yl)imino]méthyl}pyridinium, le bromure de 4-{(E)-[(1,3-dioxo-2,3-dihydro-1H-benzo[de]isoquinoléin-6-yl)imino]méthyl}-1-(2-hydroxyéthyl)pyridinium, le bromure de 2-[4-((E)-{1-[4-(diméthylamino)phényl]-2-oxo-1,2-dihydrobenzo [cd] indol-5-yl}diazényl) (éthyl) anilino] - N,N,N-triméthyléthanaminium, la 2-[(3-méthyl-1,3-benzothiazol-2(3H)-ylidène)amino]-6-nitro-1H-benzo[de]isoquinoléine-1,3(2H)-dione, la 2-(1,3-benzothiazol-2-yl)-6-nitro-1H-benzo[de]-isoquinoléine-1,3(2H)-dione, le bromure de 1-(2-hydroxyéthyl)-4-{(E)-[(14-oxo-14H-benzo[4,5]-isoquino[2,1-a]pyrimidin-9-yl)imino]méthyl}-pyridinium et la 2-{2-[(2-hydroxyéthyl)amino]-4-méthoxyphényl}-5-nitro-1H-benzo[de]isoquinoléine-1,3(2H)-dione.
